Europäisches Patentamt
European Patent Office
Office européen des brevets

(11) Veröffentlichungsnummer: **0 275 233**
**A1**

# EUROPÄISCHE PATENTANMELDUNG

(21) Anmeldenummer: **88810006.2**

(22) Anmeldetag: **08.01.88**

(51) Int. Cl.$^{4}$: **C 07 D 499/00**
A 61 K 31/43
// C07D257/04, C07F9/65

(30) Priorität: **14.01.87 US 3118**

(43) Veröffentlichungstag der Anmeldung:
**20.07.88 Patentblatt 88/29**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Lang, Marc, Dr.**
**Rue des Ormes 6**
**F-68170 Rixheim (FR)**

Die Bezeichnung der Erfindung wurde geändert (Richtlinien für die Prüfung im EPA, A-III, 7.3).

(54) **2-Heterocyclylalkyl-2-penem-Verbindungen.**

(57) Verbindungen der Formel

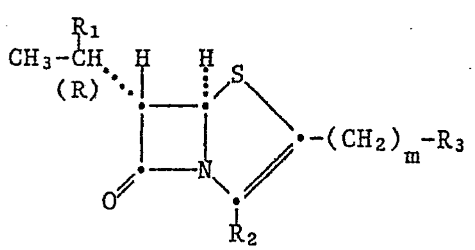

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m eine ganze Zahl von 3 bis 12 bedeutet, und Salze davon besitzen antibakterielle Aktivität. Die Verbindungen der Formel I werden nach an sich bekannten Verfahren hergestellt.

EP 0 275 233 A1

**Beschreibung**

Heterocyclyl-Verbindungen

Die Erfindung betrifft 2-Heterocyclylalkyl-2-penem-Verbindungen der Formel

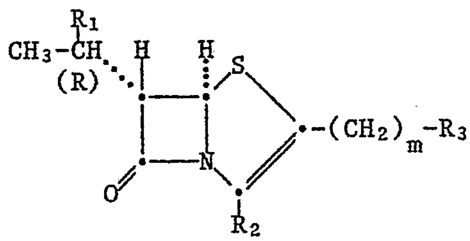

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m eine ganze Zahl von 3 bis 12 bedeutet, Salze von Verbindungen der Formel I, Verfahren zur Herstellung von Verbindungen der Formel I, pharmazeutische Präparate, die solche Verbindungen enthalten und ihre Verwendung zur Herstellung von pharmazeutischen Präparaten oder als pharmakologisch wirksame Verbindungen.

Die vor- und nachstehend verwendeten Definitionen haben im Rahmen der vorliegenden Beschreibung vorzugsweise die folgenden Bedeutungen:

Funktionell abgewandeltes Carboxyl $R_2$ ist insbesondere unter physiologischen Bedingungen spaltbares verestertes Carboxyl oder geschütztes Carboxyl $R_2'$.

Unter physiologischen Bedingungen spaltbare (d.h. metabolisierbare) veresterte Carboxylgruppen $R_2$ sind aus der Cephalosporin-, Penicillin- und Penemchemie bekannt. Geeignete Gruppen sind in erster Linie Acyloxymethoxycarbonylgruppen, worin Acyl z.B. den Rest einer organischen Carbonsäure, in erster Linie einer gegebenenfalls substituierten Niederalkancarbonsäure, bedeutet oder worin Acyloxymethyl den Rest eines Lactons bildet. Solche Gruppen sind z.B. Niederalkanoyloxymethoxycarbonyl, Aminoniederalkanoyloxymethoxycarbonyl, insbesondere α-Aminoniederalkanoyloxymethoxycarbonyl, und 4-Crotonolactonyl. Weitere unter physiologischen Bedingungen spaltbare veresterte Carboxylgruppen $R_2$ sind z.B. 5-Indanyloxycarbonyl, Phthalidyloxycarbonyl, 1-Niederalkoxycarbonyloxyniederalkoxycarbonyl, 1-Niederalkoxyniederalkoxycarbonyl oder auch 2-Oxo-1,3-dioxolen-4-ylmethoxycarbonyl, welches in 5-Stellung des Dioxolenringes gegebenenfalls durch Niederalkyl oder Phenyl substituiert ist.

Ein über ein Ringstickstoffatom gebundener monocylischer Heteroarylrest $R_3$ mit 1 bis 4 Ringstickstoffatomen ist insbesondere ein entsprechender 5-gliedriger Heteroaryl-Rest, d.h. ein entsprechender aza-, diaza-, triaza- oder tetraza-cyclischer Rest aromatischen Charakters. Entsprechende Heteroaryl-Reste $R_3$ sind beispielsweise 1-Pyrrolyl, 1-Diazolyl, wie 1-Imidazolyl oder 1-Pyrazolyl, 1,2,4-oder 1,3,4-Triazol-1-yl oder 1- oder 2-Tetrazolyl. Reste $R_3$ sind unsubstituiert oder können z.B. durch gegebenenfalls veräthertes oder veresteretes Hydroxy, z.B. Hydroxy, Niederalkoxy, Niederalkanoyloxy oder Halogen, gegebenenfalls veräthertes Mercapto, z.B. Mercapto, Niederalkylthio oder Phenylthio, Niederalkyl, Hydroxyniederalkyl, Niederalkanoyloxyniederalkyl, Carboxyniederalkyl, Carbamoylniederalkyl, Carbamoyloxyniederalkyl, Halogenniederalkyl, gegebenenfalls N-niederalkyliertes Aminoniederalkyl, z.B. Aminoniederalkyl, Niederalkylaminoniederalkyl oder Diniederalkylaminoniederalkyl, Niederalkanoylaminoniederalkyl, Amino-carboxy-niederalkyl, Sulfoniederalkyl, gegebenenfalls substituiertes Amino, z.B. Amino, Niederalkylamino, Diniederalkylamino oder Acylamino, wie Niederalkanoylamino, gegebenenfalls funktionell abgewandeltes Carboxyl oder Sulfo, z.B. Carboxyl, verestertes Carboxyl, wie Niederalkoxycarbonyl, gegebenenfalls substituiertes Carbamoyl, wie Carbamoyl, Cyano, Sulfo oder Sulfamoyl und/oder gegebenenfalls durch Niederalkyl, Niederalkoxy und/oder Halogen substituiertes Phenyl, substituiert, wie insbesondere mono- oder auch poly-, in erster Linie mono- oder disubstituiert, sein.

In der vorliegenden Beschreibung bedeutet der im Zusammenhang mit Definitionen von Gruppen bzw. Verbindungen verwendete Ausdruck "nieder", dass die entsprechenden Gruppen bzw. Verbindungen, sofern nicht ausdrücklich anders definiert, 1 bis 7, bevorzugt 1 bis 4, Kohlenstoffatome enthalten.

Niederalkanoyloxymethoxycarbonyl ist z.B. Acetoxymethoxycarbonyl oder Pivaloyloxymethoxycarbonyl.

α-Aminoniederalkanoyloxymethoxycarbonyl ist z.B. Glycyloxymethoxycarbonyl, Valyloxymethoxycarbonyl oder Leucyloxymethoxycarbonyl.

1-Niederalkoxycarbonyloxyniederalkoxycarbonyl ist z.B. Aethoxycarbonyloxymethoxycarbonyl oder 1-Aethoxycarbonyloxyäthoxycarbonyl.

1-Niederalkoxyniederalkoxycarbonyl ist z.B. Methoxymethoxycarbonyl oder 1-Methoxyäthoxycarbonyl.

Bei einer 2-Oxo-1,3-dioxolen-4-ylmethoxy-Gruppe, welche in 5-Stellung des Dioxolenringes gegebenenfalls durch Niederalkyl oder Phenyl substituiert ist, handelt es sich vor allem um eine 5-Phenyl- und in erster Linie um eine 5-Methyl-2-oxo-1,3-dioxolen-4-ylmethoxy-Gruppe.

Niederalkoxy ist z.B. Methoxy, ferner Aethoxy, n-Propyloxy, Isopropyloxy, n-Butyloxy, Isobuyloxy oder

tert.-Butyloxy, sowie n-Pentyloxy, n-Hexyloxy oder n-Heptyloxy.

Niederalkanoyloxy ist z.B. Acetyloxy oder Propionyloxy.

Halogen ist z.B. Fluor, Chlor, Brom oder Jod.

Niederalkylthio ist z.B. Methylthio, Aethylthio oder n-Propylthio.

Niederalkyl ist z.B. Methyl, Aethyl, n-Propyl, Isopropyl, n-Butyl, Isobutyl, sek.-Butyl oder tert.-Butyl, ferner n-Pentyl, n-Hexyl oder n-Heptyl.

Hydroxyniederalkyl ist z.B. Hydroxymethyl, 2-Hydroxyäthyl oder 2,3-Dihydroxypropyl.

Niederalkanoyloxyniederalkyl ist z.B. Acetoxymethyl oder 2-Acetoxyäthyl.

Carboxyniederalkyl ist z.B. Carboxymethyl, 1-Carboxy-, 2-Carboxy-oder 1,2-Dicarboxyäthyl.

Carbamoylniederalkyl ist z.B. Carbamoylmethyl oder 2-Carbamoyläthyl, während Carbamoyloxyniederalkyl z.B. Carbamoyloxymethyl oder 2-Carbamoyloxyäthyl ist.

Halogenniederalkyl ist z.B. Chlormethyl, Brommethyl, 2-Chloräthyl oder 2,2-Dichloräthyl.

Aminoniederalkyl ist z.B. Aminomethyl oder 2-Aminoäthyl, während Niederalkylaminoniederalkyl z.B. Methylaminomethyl, Aethylaminomethyl, 2-Methylaminoäthyl oder 2-Aethylaminoäthyl und Diniederalkylaminoniederalkyl z.B. Dimethylaminomethyl, 2-Dimethylaminoäthyl oder 2-Diäthylaminoäthyl ist.

Niederalkanoylaminoniederalkyl ist z.B. Acetaminomethyl, 2-Acetaminoäthyl oder Formylaminomethyl.

Amino-carboxy-niederalkyl ist z.B. 2-Amino-2-carboxy-äthyl oder auch 1-Amino-1-carboxymethyl.

Sulfoniederalkyl ist z.B. Sulfomethyl oder 2-Sulfoäthyl.

Niederalkylamino ist z.B. Methylamino, Aethylamino, n-Propylamino, Isopropylamino oder n-Butylamino, während Diniederalkylamino z.B. Dimethylamino, Diäthylamino, Di-n-propylamino oder Diisopropylamino bedeutet.

Niederalkanoylamino ist z.B. Acetylamino oder Propionylamino.

Niederalkoxycarbonyl ist z.B. Methoxycarbonyl oder Aethoxycarbonyl.

Bevorzugte unter physiologischen Bedingungen spaltbare veresterte Carboxylgruppen $R_2$ sind z.B. Niederalkanoyloxymethoxycarbonyl, z.B. Acetoxymethoxycarbonyl oder Pivaloyloxymethoxycarbonyl, und 1-Niederalkoxycarbonyloxy-niederalkoxycarbonyl, z.B. 1-Aethoxycarbonyloxyäthoxycarbonyl.

Bevorzugte Reste $R_3$ sind z.B. unsubstituiertes oder durch Niederalkyl, wie Methyl, substituiertes 1-Pyrrolyl, unsubstituiertes oder durch Niederalkyl, wie Methyl, oder Halogen, wie Chlor, substituiertes 1-Pyrazolyl, unsubstituiertes oder durch Niederalkyl, wie Methyl, Niederalkanoyloxyniederalkyl, wie Acetyloxymethyl, Halogenniederalkyl, wie Chlormethyl, Aminoniederalkyl, wie Aminomethyl oder 2-Aminoäthyl, Niederalkanoylaminoniederalkyl, wie 2-Acetylaminoäthyl, und/oder Niederalkoxy, wie Methoxy, substituiertes 1-Imidazolyl, unsubstituiertes oder durch Niederalkyl, wie Methyl, oder Niederalkoxycarbonyl, wie Methoxycarbonyl, substituiertes 1,2,4- oder 1,3,4-Triazol-1-yl, und unsubstituiertes oder durch Niederalkyl, wie Methyl, substituiertes 1- oder 2-Tetrazolyl.

In bevorzugten Verbindungen der Formel I ist m eine ganze Zahl von 3 bis 8.

Die in den Verbindungen der Formel I vorhandenen funktionellen Gruppen, wie Hydroxy-, Carboxy- oder Aminogruppen, insbesondere die Hydroxygruppe $R_1$ und die Carboxylgruppe $R_2$, sind gegebenenfalls durch Schutzgruppen geschützt, die in der Penem-, Penicillin-, Cephalosporin- und Peptidchemie verwendet werden. Solche Schutzgruppen schützen die betreffenden funktionellen Gruppen vor unerwünschten Kondensationsreaktionen, Substitutionsreaktionen und dergleichen während der Synthese der Verbindung der Formel I aus ihren Vorstufen.

Solche Schutzgruppen sind leicht, d.h. ohne dass unerwünschte Nebenreaktionen stattfinden, beispielsweise solvolytisch oder reduktiv, abspaltbar.

Schutzgruppen dieser Art sowie ihre Einführung und Abspaltung sind beispielsweise beschrieben in
J.F.W. McOmie, "Protective Groups in Organic Chemistry", Plenum Press, London, New York, 1973,
T.W. Greene, "Protective Groups in Organic Synthesis", Wiley, New York, 1981,
"The Peptides", Vol. I, Schroeder und Luebke, Academic Press, London, New York, 1965 und
Houben-Weyl, "Methoden der Organischen Chemie", Band 15/1, Georg Thieme Verlag, Stuttgart, 1974.

In Verbindungen der Formel (I) kann eine Hydroxygruppe $R_1$, ferner eine im Rest $R_3$ vorhandene Hydroxygruppe, beispielsweise durch Acylreste geschützt sein. Geeignete Acylreste sind z.B. gegebenenfalls durch Halogen substituiertes Niederalkanoyl, z.B. Acetyl, Dichloracetyl oder Trifluoracetyl, gegebenenfalls durch Nitro substituiertes Benzoyl, z.B. Benzoyl, 4-Nitrobenzoyl oder 2,4-Dinitrobenzoyl, gegebenenfalls durch Halogen substituiertes Niederalkoxycarbonyl, z.B. 2-Bromäthoxycarbonyl oder 2,2,2-Trichloräthoxycarbonyl, Niederalkenyloxycarbonyl, z.B. Allyloxycarbonyl, Niederalkenyloxyoxalyl, z.B. Allyloxyoxalyl, oder gegebenenfalls durch Nitro substituiertes Phenylniederalkoxycarbonyl, z.B. Benzyl oxycarbonyl oder 4-Nitrobenzyloxycarbonyl. Weitere geeignete Hydroxyschutzgruppen sind z.B. trisubstituiertes Silyl, wie Triniederalkylsilyl, z.B. Trimethylsilyl, Dimethyl-(2,3-dimethylbutyl)silyl oder tert.-Butyl-dimethylsilyl, und 2-Oxa- oder 2-Thiacycloalkyl mit 5 bis 7 Kohlenstoffatomen, z.B. 2-Tetrahydropyranyl oder 2-Tetrahydrofuranyl. Bevorzugt als Hydroxyschutzgruppen sind Triniederalkylsilyl, Niederalkenyloxyoxalyl und Niederalkenyloxycarbonyl.

Eine Carboxylgruppe $R_2$, ferner auch eine im Rest $R_3$ vorhandene Carboxylgruppe, ist üblicherweise in veresterter Form geschützt, wobei die Estergruppe unter schonenden Bedingungen, z.B. unter schonend reduktiven, wie hydrogenolytischen, oder schonend solvolytischen, wie acidolytischen oder insbesondere basisch oder neutral hydrolytischen Bedingungen, leicht spaltbar ist.

Solche veresterten Carboxylgruppen enthalten als veresternde Gruppen in erster Linie in 1-Stellung

verzweigte oder in 1- oder 2-Stellung geeignet substituierte Niederalkylgruppen. Geeignete in veresterter Form vorliegende Carboxylgruppen sind unter anderen gegebenenfalls durch Nitro oder Niederalkoxy, wie Methoxy, substituiertes Benzyloxycarbonyl, z.B. 4-Nitrobenzyloxycarbonyl oder 4-Methoxybenzyloxycarbonyl, Niederalkanoylmethoxycarbonyl, wie Acetonyloxycarbonyl, Benzoylmethoxycarbonyl, worin die Benzoylgruppe vorzugsweise gegebenenfalls, z.B. durch Halogen, wie Brom, substituiert ist, z.B. Phenacyloxycarbonyl, Halogenniederalkoxycarbonyl, wie 2-Halogenniederalkoxycarbonyl, z.B. 2,2,2-Trichloräthoxycarbonyl oder 2-Bromäthoxycarbonyl, Niederalkenyloxycarbonyl, z.B. Allyloxycarbonyl, oder in 2-Stellung durch Niederalkylsulfonyl, Cyano oder trisubstituiertes Silyl, wie Triniederalkylsilyl oder Triphenylsilyl, substituiertes Aethoxycarbonyl, z.B. 2-Methylsulfonyläthoxycarbonyl, 2-Cyanoäthoxycarbonyl, 2-Trimethylsilyläthoxycarbonyl oder 2-(Di-n-butyl-methyl-silyl)-äthoxycarbonyl. Bevorzugte geschützte Carboxylgruppen $R_2'$ sind die 4-Nitrobenzyloxycarbonyl-, Niederalkenyloxycarbonyl-, insbesondere Allyloxycarbonyl-, und die in 2-Stellung durch Triniederalkylsilyl, z.B. Trimethylsilyl oder Di-n-butyl-methylsilyl, substituierte Aethoxycarbonylgruppe.

Eine geschützte Aminogruppe im Rest $R_3$ kann beispielsweise in Form einer leicht spaltbaren Acylaminogruppe oder als Azidogruppe vorliegen. In einer entsprechenden Acylaminogruppe ist Acyl beispielsweise der Acylrest einer organischen Säure mit z.B. bis zu 12 Kohlenstoffatomen, insbesondere einer gegebenenfalls z.B. durch Halogen oder Phenyl, substituierten Niederalkancarbonsäure oder insbesondere eines Kohlensäurehalbesters. Solche Acylgruppen sind beispielsweise Halogenniederalkanoyl, wie 2-Halogenacetyl, insbesondere 2,2,2-Trifluor- oder 2,2,2-Trichloracetyl, Niederalkenyloxycarbonyl, z.B. Allyloxycarbonyl, gegebenenfalls in 1- oder 2-Stellung substituiertes Niederalkoxycarbonyl, wie Niederalkoxycarbony, z.B. tert.Butoxycarbonyl, gegebenenfalls substituiertes Benzyloxycarbony, z.B. 4-Nitrobenzyloxycarbonyl, -Halogenniederalkoxycarbonyl, z.B. 2,2,2-Trichloräthoxycarbonyl oder 2-Bromäthoxycarbonyl, oder 2-(trisubstituiertes Silyl)-äthoxycarbonyl, wie 2-Triniederalkylsilyläthoxycarbonyl, z.B. 2-Trimethylsilyläthoxycarbonyl oder 2-(Di-n-butylmethyl-silyl)-äthoxycarbonyl. Bevorzugte geschützte Aminogruppen sind Azido, Niederalkenyloxycarbonylamino, z.B. Allyloxycarbonylamino, und gegebenenfalls durch Nitro substituiertes Benzyloxycarbonylamino.

Salze von erfindungsgemässen Verbindungen sind in erster Linie pharmazeutisch annehmbare, nicht-toxische Salze von Verbindungen der Formel I. Solche Salze werden beispielsweise von den in Verbindungen der Formel I vorhandenen sauren Gruppen, z.B. Carboxylgruppen, gebildet und sind in erster Linie Metall- oder Ammoniumsalze, wie Alkalimetall- und Erdalkalimetall-, z.B. Natrium-, Kalium-, Magnesium- oder Kalziumsalze, sowie Ammoniumsalze mit Ammoniak oder geeigneten organischen Aminen, wie Niederalkylaminen, z.B. Triäthylamin, Hydroxyniederalkylaminen, z.B. 2-Hydroxyäthylamin, Bis-(2-hydroxyäthyl)-amin oder Tris-(2-hydroxyäthyl)-amin, basischen aliphatischen Estern von Carbonsäuren, z.B. 4-Aminobenzoesäure-2-di äthylaminoäthylester, Niederalkylenaminen, z.B. 1-Aethyl-piperidin, Cycloalkylaminen, z.B. Dicyclohexylamin, oder Benzylaminen, z.B. N,N'-Dibenzyläthylendiamin, Dibenzylamin oder N-Benzyl-β-phenäthylamin. Verbindungen der Formel I mit einer basischen Gruppe, z.B. mit einer Aminogruppe, können Säureadditionssalze, z.B. mit anorganischen Säuren, wie Salzsäure, Schwefelsäure oder Phosphorsäure, oder mit geeigneten organischen Carbon- oder Sulfonsäure, z.B. Essigsäure, Bernsteinsäure, Fumarsäure, Maleinsäure, Weinsäure, Oxalsäure, Zitronensäure, Benzoesäure, Mandelsäure, Aepfelsäure, Ascorbinsäure, Methansulfonsäure oder 4-Toluolsulfonsäure bilden. Verbindungen der Formel I mit einer sauren und mit einer basischen Gruppe können auch in Form von inneren Salze, d.h. in zwitterionischer Form, vorliegen.

Zur Isolierung oder Reinigung können auch pharmazeutisch ungeeignete Salze Verwendung finden. Zur therapeutischen Anwandung gelangen nur die pharmazeutisch annehmbaren, nicht-toxischen Salze, die deshalb bevorzugt sind.

Die Verbindungen der Formel I weisen wertvolle pharmakologische Eigenschaften auf oder können als Zwischenprodukte zur Herstellung von solchen Verbindungen mit wertvollen pharmakologischen Eigenschaften verwendet werden. Verbindungen der Formel I, worin $R_1$ Hydroxy ist, $R_2$ Carboxyl oder eine unter physiologischen Bedingungen spaltbare veresterte Carboxylgruppe darstellt, $R_3$ und m die unter Formel I angegebenen Bedeutungen haben, und pharmakologisch annehmbare Salze von solchen Verbindungen mit salzbildenden Gruppen haben antibakterielle Wirkungen. Beispielsweise sind sie in vitro gegen grampositive umd gramnegative Kokken, z.B. Staphylococcus aureus, Streptococcus pyogenes, Streptococcus pneumoniae, Streptococcus faecalis, Neisseria meningitidis und Neisseria gonorrhoeae in minimalen Konzentrationen von ca. 0,01 bis 4 µg/ml wirksam. In vivo, bei der systemischen Infektion der Maus, z.B. durch Streptococcus pneumoniae, ergeben sich bei subkutaner oder oraler Applikation $ED_{50}$-Werte von ca. 1 bis ca. 20 mg/kg. Die erfindungsgemässen Verbindungen, worin m eine ganze Zahl von 3 bis 5 bedeutet und $R_1$, $R_2$ und $R_3$ die unter Formel I angegebenen Bedeutungen haben, sind ausserdem gegen gramnegative Stäbchenbakterien, z.B. Haemophilus influenzae, Escherichia coli und Proteus spec., und Anaerobier, z.B. Bacteroides spec., wirksam, in vitro beispielsweise in minimalen Konzentrationen von ca. 0,02 bis ca. 32 µg/ml.

Die Verbindungen der vorliegenden Erfindung erweisen sich im Test mit dem aus menschlichen Nieren isolierten Enzym Dehydropeptidase als bemerkenswert stabil und weisen Halbwertszeiten von ca. 1,8 bis ca. 4 Stunden auf.

In der folgenden Tabelle sind die antibakteriellen Aktivitäten (MIC, in µg/ml) und die Stabilitäten gegenüber renaler Dehydropeptidase (in Halbwertszeiten t1/2) von erfindungsgemässen Verbindungen sowie von homologen Verbindungen aus dem Stand der Technik zusammengefasst. Im einzelnen handelt es sich bei den getesteten Verbindungen um die folgenden:

Verbindung 1*: (5R,6S)-6-Hydroxymethyl-2-[4-(tetrazol-1-yl)-butyl]-2-penem-3-carbonsäure (Vergleichssubstanz aus EP 110826),

Verbindung 2*: (5R,6S)-6-[(1'R)-1'-Hydroxyäthyl]-2-[2-(tetrazol-1-yl)-äthyl]-2-penem-3-carbonsäure (Vergleichssubstanz aus EP 110826),

Verbindung 3: (5R,6S)-6-[(1'R)-1'-Hydroxyäthyl]-2-[3-(tetrazol-1-yl)-propyl]-2-penem-3-carbonsäure,

Verbindung 4: (5R,6S)-6-[(1'R)-1'-Hydroxyäthyl]-2-[4-(tetrazol-1-yl)-butyl]-2-penem-3-carbonsäure,

Verbindung 5: (5R,6S)-6-[(1'R)-1'-Hydroxyäthyl]-2-[5-(tetrazol-1-yl)-pentyl]-2-penem-3-carbonsäure,

Verbindung 6: (5R,6S)-6-[(1'R)-1'-Hydroxyäthyl]-2-[7-(tetrazol-1-yl)-heptyl]-2-penem-3-carbonsäure und

Verbindung 7: (5R,6S)-6-[(1'R)-1'-Hydroxyäthyl]-2-[10-(tetrazol-1-yl)-decyl]-2-penem-3-carbonsäure.

Tabelle: Antibakterielle Aktivität in vitro gegen grampositive und gramnegative Kokken und Stabilität gegenüber Dehydropeptidase aus menschlichen Nieren

| Mikroorganismen | Verbindung in vitro MIC (µg/ml) | | | | | | |
|---|---|---|---|---|---|---|---|
| | 1* | 2* | 3 | 4 | 5 | 6 | 7 |
| Staph. aureus 10 B | 0,1 | 0,02 | 0,02 | 0,01 | 0,02 | 0,02 | 0,02 |
| Staph. aureus 2999 | 0,1 | 0,02 | 0,02 | 0,02 | 0,02 | 0,02 | 0,02 |
| Staph. aureus A325 MR | n.b. | n.b. | 1 | 0,5 | 2 | 2 | 1 |
| Staph. aureus Wood 46 | 0,1 | 0,02 | 0,02 | 0,01 | 0,02 | 0,02 | 0,02 |
| Strep. pyogenes Aronson | 0,2 | 0,02 | 0,01 | 0,01 | 0,02 | 0,02 | 0,01 |
| Strep. pneumoniae III/84 | 0,05 | <0,01 | <0,01 | <0,01 | <0,01 | <0,01 | <0,01 |
| Strep. pneumoniae Q 13 | n.b | n.b | 0,5 | 0,2 | 0,2 | 0,1 | 0,1 |
| Strep. faecalis D3 | 16 | 8 | 4 | 2 | 2 | 1 | 1 |
| Neiss. meningitidis 1316 | 0,05 | 0,01 | 0,01 | <0,01 | <0,01 | <0,01 | 0,1 |
| Neiss. gonorrhoeae 1317 | 0,01 | <0,01 | <0,01 | <0,01 | <0,01 | <0,01 | 0,01 |
| | t 1/2 (Std.) | | | | | | |
| Dehydropeptidase | 4,3 | 0,12 | 3,75 | 3,83 | 3,30 | 1,84 | 1,2 |

(Staph.: Staphylococcus; Strep.: Streptococcus; Neiss.: Neisseria; n.b.: nicht bestimmt).

Alle erfindungsgemässen Verbindungen weisen gegenüber der Vergleichsverbindung 1* erheblich niedrigere MIC-Werte (Faktor 4-20) auf. In Vergleich zur Vergleichsverbindung 2* besitzen die Verbindungen der vorliegenden Erfindung bei etwa gleicher oder besserer antibakterieller Aktivität gegen grampositive und -negative Kokken eine erheblich grössere Stabilität gegenüber dem desaktivierenden Nierenenzym Dehydropeptidase und damit eine längere Verweildauer und antibakterielle Wirkungsdauer in vivo.

In Gegensatz zu den Verbindungen der Formel I mit kürzerer AlkylenSeitenkette (m = 3-5) und allen strukturell vergleichbaren Penem-Verbindungen aus dem Stand der Technik, z.B. den oben genannten Vergleichssubstanzen 1* und 2*, weisen die erfindungsgemässen Penem-Verbindungen mit längerer Alkylen-Seitenkette (m = 6-12) darüberhinaus ein bemerkenswert selektives antibakterielles Spektrum auf. Ihr Wirkungsspektrum ist im wesentlichen auf grampositive und gramnegative Kokken beschränkt, wobei besonders die hohe Aktivität gegen den bekannten Problemkeim Streptococcus faecalis zu erwähnen ist, während die Wirkung gegen gramnegative Stäbchenbakterien gering oder nicht vorhanden ist. Diese Eigenschaft macht die genannten Peneme besonders geeignet für die konische Behandlung schwerer Staphylococcus- und Streptococcus-Infektionen, insbesondere in solchen Fällen, in denen die Behandlung mit üblichen Breitbandantibiotika nicht erfolgversprechend erscheint, z.B. wegen zu geringer Wirkung gegen resistente Stämme, oder nicht angezeigt ist, beispielsweise wenn die übrige körpereigene Flora (z.B. Darmflora) geschont werden soll oder muss. Wegen ihrer guten Verträglichkeit und geringeren Toxizität können die genannten Peneme auch in besonders kritischen Fällen, in denen die Verabreichung ähnlich spezifischer Antibiotika, wie Vancomycin oder Lincomycin, wegen zu grosser Toxizität nicht verantwortet werden kann, z.B. bei eingeschränkter Nierenfunktion, eingesetzt werden.

Die neuen Verbindungen können dementsprechend als oral oder parenteral applizierbare antibakterielle

Antibiotika, z.B. in Form von entsprechenden pharmazeutischen Präparaten, zur Behandlung von Infektionen, insbesondere solchen, die durch grampositive oder gramnegative Kokken verursacht werden, Verwendung finden.

Verbindungen der Formel I, worin mindestens eine der vorhandenen funktionellen Gruppen in geschützter Form vorliegt, können als Zwischenprodukte zur Herstellung der oben genannten pharmakologisch wirksamen Verbindungen der Formel I verwendet werden.

Die Erfindung betrifft insbesondere Verbindungen der Formel I, worin $R_1$ Hydroxy oder geschütztes Hydroxy bedeutet, $R_2$ Carboxyl, unter physiologischen Bedingungen spaltbares verestertes Carboxyl oder geschütztes Carboxyl ist, $R_3$ einen über ein Ringstickstoffatom gebundenen monocylischen 5-gliedrigen Heteroarylrest mit 1 bis 4 Ringstickstoffatomen darstellt, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Carbamoylnieder alkyl, Carbamoyloxyniederalkyl, Halogenniederalkyl, Aminoniederalkyl, Niederalkylaminoniederalkyl, Diniederalkylaminoniederalkyl, Niederalkanoylaminoniederalkyl, Amino-carboxy-niederalkyl, Sulfoniederalkyl, Amino, Niederalkylamino, Diniederalkylamino, Niederalkanoylamino, Carboxyl, Niederalkoxycarbonyl, Carbamoyl, Cyano, Sulfo, Sulfamoyl und/oder gegebenenfalls durch Niederalkyl, Niederalkoxy und/oder Halogen substituiertes Phenyl substituiert ist, und m eine ganze Zahl von 3 bis 12 bedeutet, und Salze von Verbindungen der Formel I.

Die Erfindung betrifft hauptsächlich Verbindungen der Formel I, worin $R_1$ Hydroxy ist, $R_2$ Carboxyl oder unter physiologischen Bedingungen spaltbares verestertes Carboxyl bedeutet, $R_3$ unsubstituiertes oder durch Niederalkyl substituiertes 1-Pyrrolyl, unsubstituiertes oder durch Niederalkyl oder Halogen substituiertes 1-Pyrazolyl, unsubstituiertes oder durch Niederalkyl, Niederalkanoyloxyniederalkyl, Halogenniederalkyl, Aminoniederalkyl, Niederalkanoylaminoniederalkyl und/oder Niederalkoxy substituiertes 1-Imidazolyl, unsubstituiertes oder durch Niederalkyl oder Niederalkoxycarbonyl substituiertes 1,2,4- oder 1,3,4-Triazol-1-yl, oder unsubstituiertes oder durch Niederalkyl substituiertes 1- oder 2-Tetrazolyl bedeutet und m eine ganze Zahl von 3 bis 10 ist, und Salze von Verbindungen der Formel I.

Die Erfindung betrifft in erster Linie Verbindungen der Formel I, worin $R_1$ Hydroxy ist, $R_2$ Carboxyl oder unter physiologischen Bedingungen spaltbares verestertes Carboxyl bedeutet, $R_3$ 1-Tetrazolyl ist und m eine ganze Zahl von 3 bis 8 ist, und Salze von Verbindungen der Formel I.

Die Erfinding betrifft in erster Linie die in den Beispielen genannten Verbindungen der Formel I und deren Salze, insbesondere deren pharmazeutisch annehmbare Salze.

Die Verbindungen der vorliegenden Erfindung können nach an sich bekannten Verfahren hergestellt werden.

Die neuen Verbindung werden z.B. hergestellt, indem man eine Ylid-Verbindung der Formel

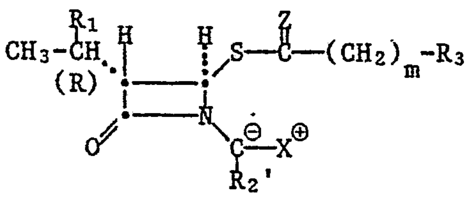

(II),

worin $R_1$,m und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

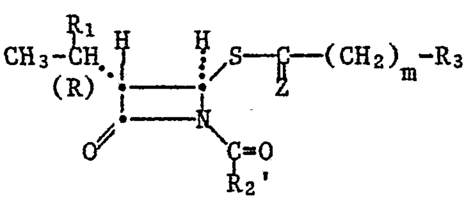

(III),

worin $R_1$, m und $R_3$ die unter Formel I und $R_2'$und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppen in die freien funktionellen Gruppen überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in eine Salz oder ein erhältliches Salz in die freie Verbindung oder in ein anderes Salz überführt.

In den Ausgangsverbindungen der Formeln II und III sind funktionelle Gruppen, wie eine freie Hydroxygruppe $R_1$ sowie weitere im Rest $R_3$ enthaltene funktionelle Gruppen, vorzugsweise durch konventionelle Schutzgruppen, z.B. durch die oben genannten, geschützt.

Cyclisierung der Verbindung der Formel II

Die Gruppe $X^{\oplus}$ im Ausgangsmaterial der Formel II ist eine der bei Wittig-Kondensationsreaktionen gebräuchlichen Phosphonio- oder Phosphonogruppen, insbesondere eine Triaryl-, z.B. Triphenyl-, oder Triniederalkyl-, z.B. Tri-n-butylphosphoniogruppe, oder eine durch Niederalkyl, z.B. Aethyl, zweifach veresterte Phosphonogruppe, wobei das Symbol $X^{\oplus}$ für den Fall der Phosphonogruppe zusätzlich das Kation einer starken Base, insbesondere ein geeignetes Metall-, wie Alkalimetallion, z.B. das Lithium-, Natrium- oder Kaliumion, umfasst. Bevorzugt als Gruppe $X^{\oplus}$ sind einerseits Triphenylphosphonio und andererseits Diäthylphosphono zusammen mit einem Alkalimetall-, z.B. dem Natriumion.

Der Ringschluss kann spontan, d.h. bei der Herstellung der Ausgangsstoffe, oder durch Erwärmen, z.B. in einem Temperaturbereich von etwa 30° C bis 160° C, vorzugsweise von etwa 50° C bis etwa 110° C, erfolgen. Die Reaktion wird vorzugsweise in einem geeigneten inerten Lösungsmittel, wie in einem aliphatischen, cycloaliphatischen oder aromatischen Kohlenwasserstoff, z.B. Cyclohexan, Benzol oder Toluol, einem halogenierten Kohlenwasserstoff, z.B. Methylenchlorid, einem Aether, z.B. Diäthyläther, einem cyclischen Aether, z.B. Dioxan oder Tetrahydrofuran, einem Carbonsäureamid, einem Diniederalkylsulfoxid, oder einem Niederalkanol oder in einem Gemisch davon, und, falls notwendig, in einer Inertgas-, z.B. Stickstoffatmosphäre, durchgeführt.

Cyclisierung der Verbindung der Formel III

Eine organische Verbindung des dreiwertigen Phosphors leitet sich z.B. von phosphoriger Säure ab und ist insbesondere ein Ester derselben mit einem Niederalkanol, z.B. Methanol oder Aethanol, und/oder einer gegebenfalls substituierten aromatischen Hydroxy verbindung, z.B. Phenol oder Brenzcatechin, oder ein Amidester derselben der Formel $P(OR_a)_2N(R_b)_2$, worin $R_a$ und $R_b$ unabhängig voneinander Niederalkyl, z.B. Methyl, oder Aryl, z.B. Phenyl, bedeuten. Bevorzugte Verbindungen des dreiwertigen Phosphors sind Triniederalkylphosphite, z.B. Trimethylphosphit oder Triäthylphosphit.

Die Reaktion wird vorzugsweise in einem inerten Lösungsmittel, wie einem aromatischen Kohlenwasserstoff, z.B. Benzol oder Toluol, einem Aether, z.B. Dioxan oder Tetrahydrofuran, oder einem halogenierten Kohlenwasserstoff, z.B. Methylenchlorid oder Chloroform, bei einer Temperatur von etwa 20° bis 140°C, bevorzugt von etwa 20° bis etwa 110°C, durchgeführt, wobei man 1 Moläquivalent einer Verbindung der Formel III mit mindestens 2 Moläquivalenten der Phosphorverbindung umsetzt. Vorzugsweise legt man die Verbindung der Formel III in einem inerten Lösungsmittel vor und tropft die Phosphorverbindung, vorzugsweise in dem gleichen inerten Lösungsmittel gelöst, hinzu.

In einer bevorzugten Ausführungsform des Verfahrens stellt man das Ausgangsmaterial der Formel III wie weiter unten angegeben her und setzt es ohne Isolierung aus dem Reaktionsgemisch mit der organischen Verbindung des dreiwertigen Phsophors um, wobei die Endprodukte der Formel I entstehen.

Verfahrensgemäss erhältliche Verbindungen der Formel I können in an sich bekannter Weise in andere Verbindungen der Formel I überführt werden.

So können in einer erhältlichen Verbindung der Formel I, worin eine oder mehrere funktionelle Gruppen geschützt sind, diese, z.B. geschützte Carboxyl-, Hydroxy- und/oder Aminogruppen, in an sich bekannter Weise mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig freigesetzt werden.

In einer verfahrensgemäss erhältlichen Verbindung der Formel I, worin $R_2$ eine geschützte Carboxylgruppe bedeutet und/oder worin der Rest $R_3$ geschütztes Carboxyl als Substituenten enthält, kann die geschützte Carboxylgruppe in an sich bekannter Weise freigesetzt werden. So kann man in 2-Stellung durch eine trisubstituierte Silylgruppe substituiertes Aethoxycarbonyl z.B. durch Behandeln mit einer Carbonsäure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, gespalten werden. Ferner kann man geeigne substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zinn, üblicherweise in Gegenwart eines Wasserstoff-abgebenden Mittels, das zusammen mit dem Metall nascierenden Wasserstoff zu erzeugen vermag, wie einer geeigneten Carbonsäure, z.B. einer gegebenenfalls, z.B. durch Hydroxy, substituierten Niederalkancarbonsäure, z.B. Essigsäure, oder eines Alkohols oder Thiols, wobei man vorzugsweise Wasser zugibt, in freies Carboxyl überführen. Die Abspaltung einer Allylschutzgruppe kann z.B. durch Umsetzung mit einer Verbindung des Palladiums, z.B. Tetrakis-(triphenylphosphin)-palladium, gegebenenfalls in Gegenwart von Triphenylphosphin und unter Zusatz eines Allylgruppenakzeptors, wie einer Carbonsäure, z.B. 2-Aethylhexansäure, oder eines Salzes davon oder Tributylzinnhydrid oder Dimedon, erfolgen. Durch Behandeln mit einem reduzierenden Metall oder Metallsalz, wie oben beschrieben, kann man auch 2-Halogenniederalkoxycarbonyl (gegebenenfalls nach Umwandlung einer 2-Bromniederalkoxycarbonylgruppe in eine entsprechende 2-Jodniederalkoxycarbonylgruppe) oder Benzoylmethoxycarbonyl in freies Carboxyl umwandeln, wobei Benzoylmethoxycarbonyl auch durch Behandeln mit einem nucleophilen, vorzugsweise salzbildenden Reagenz, wie Natriumthiophenolat oder Natriumjodid, gespalten werden kann. Substituiertes 2-Silyläthoxycarbonyl kann auch durch Behandeln mit einem das Fluoridanion liefernden Salz

der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammoniumfluorid, z.B. Tetrabutylammoniumfluorid, in freies Carboxyl überführt werden. Eine in 2-Stellung durch Niederalkylsulfonyl oder Cyano substituierte Niederalkoxycarbonylgruppe kann z.B. durch Behandeln mit einem basischen Mittel, wie einem Alkalimetall- oder Erdalkalimetallhydroxid oder -carbonat, z.B. Natrium-oder Kaliumhydroxid oder Natrium- oder Kaliumcarbonat, in freies Carboxyl übergeführt werden.

Andererseits können auch Verbindungen der Formel I, worin $R_2$ Carboxyl bedeutet, in Verbindungen der Formel I überführt werden, worin $R_2$ eine unter physiologischen Bedingungen spaltbare veresterte Carboxylgruppe darstellt. Solche Ester können z.B. durch Umsetzung eines gegebenenfalls in situ hergestellten Salzes der Säure mit einem reaktionsfähigen Ester eines entsprechenden Alkohols und einer starken anorganischen Säure, wie Schwefelsäure, oder einer starken organischen Sulfonsäure, wie 4-Toluolsulfonsäure, hergestellt werden. Ferner kann man in Verbindungen der Formel I, die eine in veresterter Form geschützte Carboxylgruppe enthalten, die Carboxylschutzgruppe wie oben beschrieben abspalten und eine entstandene Verbindung der Formel I mit einer freien Carboxylgruppe oder ein Salz davon durch Umsetzen mit dem reaktionsfähige Ester eines entsprechenden Alkohols in eine Verbindung der Formel I überführen, worin $R_2$ eine unter physiologischen Bedingungen spaltbare veresterte Carboxylgruppe darstellt.

In verfahrensgemäss erhältlichen Verbindungen der Formel I, worin $R_1$ geschütztes Hydroxy bedeutet und/oder gegebenenfalls der Rest $R_3$ geschütztes Hydroxyl als Substituenten enthält, kann die geschützte Hydroxygruppe in an sich bekannter Weise in die freie Hydroxygruppe überführt werden. Beispielsweise wird eine durch eine geeignete Acylgruppe oder eine organische Silylgruppe geschützte Hydroxygruppe wie eine entsprechend geschützte Aminogruppe freigesetzt (s.u.); eine Triniederalkylsilylgruppe kann z.B. auch mit Tetrabutylammoniumfluorid und Essigsäure gespalten werden (unter diesen Bedingungen werden durch trisubstituiertes Silyläthoxy geschützte Carboxygruppen nicht gespalten).

In einer erfindungsgemäss erhältlichen Verbindung der Formel I mit einer geschützten Aminogruppe kann diese in an sich bekannter Weise, z.B. je nach Art der Schutzgruppe, vorzugsweise mittels Solvolyse oder Reduktion, in die freie Aminogruppe übergeführt werden. Beispielsweise kann 2-Halogenniederalkoxycarbonylamino (gegebenenfalls nach Umwandlung einer 2-Bromniederalkoxycarbonylaminogruppe in eine 2-Jodniederalkoxycarbonylaminogruppe) und 4-Nitrobenzyloxycarbonylamino durch Behandeln mit einem geeigneten chemischen Reduktionsmittel, wie Zink in Gegenwart einer geeigneten Carbonsäure, wie wässriger Essigsäure, oder katalytisch mit Wasserstoff in Gegenwart eines Palladiumkatalysators oder durch Behandeln mit einem Alkalimetall-, z.B. Natriumdithionit gespalten werden. Gegebenenfalls substituiertes Benzyloxycarbonylamino kann z.B. mittels Hydrogenoylse, d.h. durch Behandeln mit Wasserstoff in Gegenwart eines geeigneten Hydrierkatalysators, wie eines Palladiumkatalysators, und Allyloxycarbonylamino durch Umsetzen mit einer Verbindung des Palladiums, z.B. Tetrakis(triphenylphosphin)palladium, gegebenenfalls in Gegenwart von Triphenylphosphin und in Gegenwart eines Allylgruppenakzeptors, wie einer Carbonsäure, z.B. 2-Aethylhexansäure, oder eines Salzes davon oder Tributylzinnhydrid oder Dimeon, gespalten werden. Eine durch 2-Halogenniederalkanoyl, z.B. 2-Chloracetyl, geschützte Aminogruppe kann durch Behandeln mit Thioharnstoff in Gegenwart einer Base oder mit einem Salz, wie einem Alkalimetall-Salz, des Thioharnstoffs und anschliessender Solvolyse wie Alkoholyse oder Hydrolyse des entstandenen Kondensationsproduktes freigesetzt werden. Eine durch 2-substituiertes Silyläthoxycarbonyl geschützte Aminogruppe kann durch Behandeln mit einem Fluoridanion liefernden Salz der Fluorwasserstoffsäure, wie einem Alkalimetallfluorid, z.B. Natriumfluorid, in Anwesenheit eines makrocyclischen Polyäthers ("Kronenäther") oder mit einem Fluorid einer organischen quaternären Base, wie Tetraniederalkylammonium fluorid, z.B. Tetraäthylammoniumfluorid, in die freie Aminogruppe übergeführt werden. Eine in Form einer Azidogruppe geschützte Aminogruppe wird z.B. durch Reduktion in freies Amino überführt, beispielsweise durch katalytische Hydrierung mit Wasserstoff in Gegenwart eines Hydrierkatalysators wie Platinoxid oder Palladium, oder durch Behandeln mit Zink in Gegenwart einer Säure, wie Essigsäure.

In Verbindungen der Formel I kann man weiterhin einer Rest $R_3$ in einen anderen Rest $R_3$ überführen.

So kann beispielsweise in Verbindungen der Formel I, worin der Rest $R_3$ durch eine Carboxylgruppe substituiert ist, diese Carboxylgruppe nach an sich bekannten Verfahren in eine funktionell abgewandelte Carboxylgruppe, wie in eine veresterte Carboxylgruppe, überführt werden. Beispielsweise erhält man durch Umsetzen einer Verbindung der Formel I, worin der Rest $R_3$ durch Carboxyl substituiert ist, mit einem Niederalkanol eine Verbindung der Formel I, worin $R_3$ durch Niederalkoxycarbonyl substituiert ist, wobei man vorzugsweise in Gegenwart eines geeigneten Kondensationsmittels, z.B. eines Carbodiimids, arbeitet oder das entstehende Wasser durch azeotrope Destillation entfernt. Andererseits kann man Carboxylgruppen an Resten $R_3$ auch in reaktionsfähige funktionelle Derivate, wie gemischte Anhydride, z.B. Säurehalogenide, oder aktivierte Ester, überführen und diese durch Umsetzen mit einem Alkohol, z.B. einem Niederalkanol, in entsprechend veresterte Carboxylgruppen umwandeln, wobei man bei Verwendung von gemischten Anhydriden vorzugsweise in Gegenwart eines säurebindenden Mittels, wie eines aromatischen oder tertiären aliphatischen Amins oder eines Alkalimetalloder Erdalkalimetallcarbonate, arbeitet.

In Verbindungen der Formel I, worin der Rest $R_3$ durch Amino substituiert ist, kann die Aminogruppe in eine substituierte Aminogruppe, z.B. eine Niederalkylamino-, Diniederalkylamino- oder Niederalkanoylaminogruppe, überführt werden. Die Ueberführung in eine Niederalkylamino- oder Diniederalkylaminogruppe erfolgt beispiels weise durch Reaktion mit einem reaktionsfähigen veresterten Niederalkanol, beispielsweise einem Niederalkylhalogenid oder -sulfonat, in Gegenwart eines basischen Kondensationsmittels, wie eines

Hydroxids oder Carbonats eines Alkali- oder Erdalkalimetalls oder einer heteroaromatischen Stickstoffbase, z.B. Pyridin. In analoger Weise kann Amino durch Behandeln mit dem reaktionsfähigen funktionellen Derivat einer Niederalkancarbonsäure, z.B. dem entsprechenden Carbonsäurehalogenid, in Niederalkanoylamino umgewandelt werden.

Salze von Verbindungen der Formel I mit salzbildenden Gruppen können in an sich bekannter Weise hergestellt werden. So kann man Salze von Verbindungen der Formel I mit einer freien Carboxyl- oder Sulfogruppe z.B. durch Behandeln mit Metallverbindung, wie Alkalimetallsalzen von geeigneten organischen Carbonsäure, z.B. dem Natriumsalz der α-Aethylcapronsäure, oder mit anorganischen Alkalioder Erdalkalimetallsalzen, z.B. Natriumhydrogencarbonat, oder mit Ammoniak oder mit einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer geeigneten Säure oder einem geeigneten Anionenaustauscherreagenz. Innere Salze von Verbindungen der Formel I können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze z.B. durch Behandeln mit geeigneten Säuren und Säureadditionssalze z.B. durch Behandeln mit einem geeigneten basischen Mittel.

Bei allen nachträglichen Umwandlungen erhaltener Verbindungen der Formel I werden solche Reaktionen bevorzugt, die unter gemässigt alkalischen oder insbesondere neutralen Bedingungen erfolgen.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird. Ferner können Ausgangsstoffe in Form von Derivaten verwendet oder in situ, gegebenenfalls unter den Reaktionsbedingungen, hergestellt werden.

Die Ausgangsverbindungen der Formeln II und III können, wie in dem folgenden Reaktionsschema I angegeben, hergestellt werden:

<u>Reaktionsschema I</u>

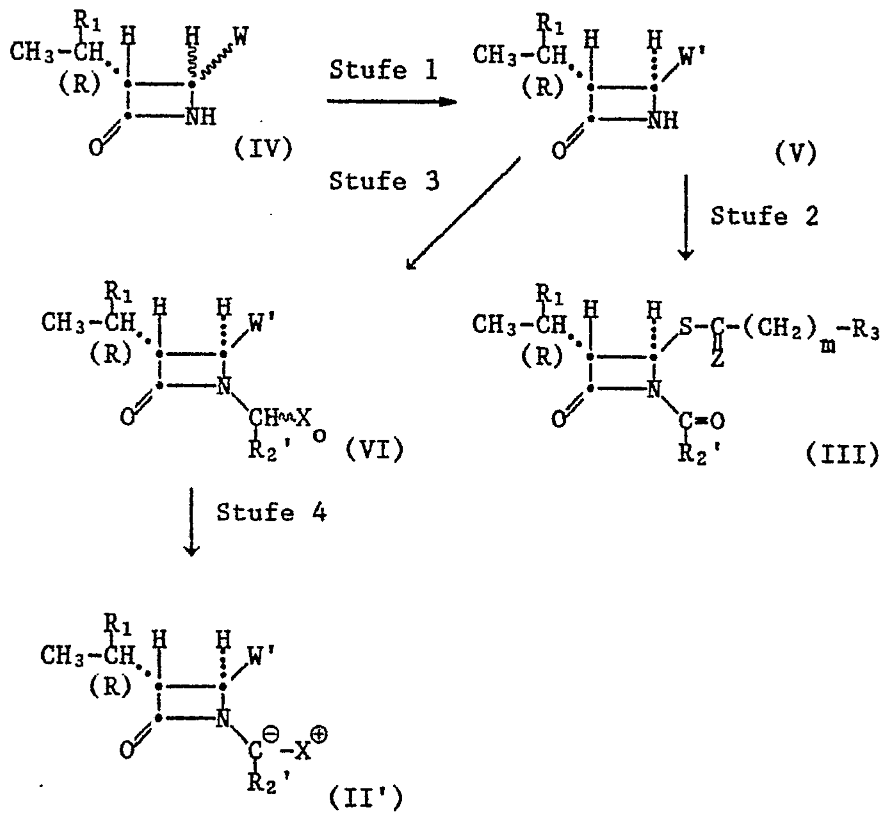


In den Verbindungen der Formel V, VI und II'steht W' für den Rest -S-C(=Z)-$(CH_2)_m$-$R_3$ oder für Triphenylmethylthio oder Niederalkanoylthio.

<u>Stufe 1</u>

Geeignete Ausgangsverbindungen der Formel IV, worin W ein durch nucleophile Reaktion leicht austauschbarer Rest, z.B. Niederalkanoyloxy, wie Acetyloxy, oder Sulfonyloxy $R_0$-$SO_2$-, worin $R_0$ z.B. gegebenenfalls durch Hydroxy substituierten Niederalkyl, wie Methyl, tert.Butyl oder 2-Hydroxyäthyl, ist, sind beispielsweise aus der veröffentlichten Europäischen Patentanmeldung Nr. 82113, der Deutschen

Offenlegungsschrift Nr. 3 224 055 und der Deutschen Offenlegungsschrift Nr. 3 013 997 bekannt oder können in dazu analoger Weise hergestellt werden.

Eine den Rest -S-C(=Z)-$(CH_2)_m$-$R_3$ einführende Verbindung ist beispielswiese eine Säure der Formel $R_3$-$(CH_2)_m$-C(=Z)-SH oder insbesondere ein Salz, z.B. ein Alkalimetallsalz, wie das Natriumoder Kaliumsalz, davon. Die Substitution kann in einem organischen Lösungsmittel, wie in einem Niederalkanol, einem Niederalkancarbonsäureamid, einem cyclischen Aether, oder in einem ähnlichen inerten Lösungsmittel bei Raumtemperatur oder bei etwas erhöhter oder erniedrigter Temperatur, z.B. bei etwa 0° bis etwa 40°C, durchgeführt werden. Die Einführung eines Triphenylmethylthio- oder Niederalkanoylthio-Restes W' erfolgt in analoger Weise durch Umsetzen mit einem Alkalimetallsalz, z.B. dem Natriumsalz, einer Thioniederalkancarbonsäure, z.B. Thioessigsäure, oder des Triphenylmethylmercaptans.

Stufe 2

Eine Ausgangsverbindung der Formel (III) wird erhalten, indem man ein Azetidinon der Formel (V), in der W' für den Rest -S-C(=Z)-$(CH_2)_m$-$R_3$ steht, mit einer Säure der Formel $R_2$'-COOH oder insbesondere einem reaktionsfähigen Derivat, wie einem Ester oder Säurehalogenid, z.B. dem Säurechlorid, davon bei einer Temperatur von -50° bis 80°C, bevorzugt bei -20° bis 0°C, in einem inerten Lösungsmittel, wie einem der bei der Umsetzung von Verbindungen der Formel (III) zu Verbindungen der Formel I genannten, behandelt. Bei Verwendung eines Säurehalogenids arbeitet man vorzugsweise in Gegenwart eines säurebindenden Mittels, wie eines tertiären alipha tischen Amins, eines aromatischen Amins, oder insbesondere eines Alkalimetall- oder Erdalkalimetallcarbonats oder -hydrogencarbonats.

Verbindungen der Formel (V), worin W' für Triphenylmethylthio oder Niederalkanoylthio steht, können in die Ausgangsverbindungen der Formel V, worin W' für den Rest -S-C(=Z)-$(CH_2)_m$-$R_3$ steht, überführt werden, indem man sie in Gegenwart einer Base, z.B. Pyridin oder Tri-n-butylamin, in einem geeigneten Lösungsmittel, z.B. Diäthyläther oder Methanol, mit einem Salz der Formel MA, worin M für ein Uebergangsmetallkation, insbesondere für das Silberkation, steht, und A ein gebräuchliches Anion darstellt, welches die Löslichkeit des Salzes MA in dem gewählten Lösungsmittel begünstigt, z.B. das Nitrat-, Acetat- oder Fluorid-Anion, umsetzt, und das entstandene Salz der Formel

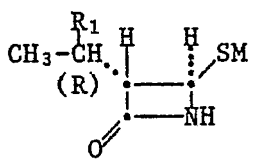

(V'),

mit einem den Rest $R_3$-$(CH_2)_m$-C(=Z)- einführenden Acylierungsmittel, z.B. mit der Säure $R_3$-$(CH_2)_m$-C(=Z)-OH oder einem reaktionsfähigen funktionellen Derivat, wie einem Säurehalogenid, z.B. dem Chlorid oder Bromid, Azid oder Anhydrid davon, behandelt. Die Acylierung erfolgt,wenn ein reaktionsfähiges funktionelles Derivat der Säure der Formel $R_3$-$(CH_2)_m$-C(=Z)-OH, z.B. das Säurechlorid, eingesetzt wird, in einem inerten Lösungsmittel, wie einem chlorierten Kohlenwasserstoff, oder einem Aether, bei Raumtemperatur oder unter Erhitzen oder Kühlen, z.B. in einem Temperaturbereich von ca. -50° bis ca. +60°C, insbesondere bei ca. -30° bis ca. +20°C.

Stufe 3

Verbindungen der Formel VI, worin $X_0$ für eine reaktionsfähige veresterte Hydroxygruppe, insbesondere für Halogen, z.B. Chlor oder Brom, steht, werden hergestellt, indem man eine Verbindung der Formel V mit einer Glyoxylsäure-Verbindung der Formel $R_2$'-CHO oder einem geeigneten Derivat davon, wie einem Hydrat, Hemihydrat oder Halbacetal, z.B. einem Halbacetal mit einem Niederalkanol, z.B. Methanol oder Aethanol, umsetzt, und in einer erhaltenen Verbindung der Formel VI, worin $X_0$ für Hydroxy steht, die Hydroxygruppe in eine reaktionsfähige veresterte Hydroxygruppe umwandelt. Die Verbindungen der Formel VI werden üblicherweise als Gemisch der beiden Isomeren [bezüglich der Gruppierung -CH($R'_2$)∿$X_0$] erhalten.

Die Anlagerung der Glyoxylsäureester-Verbindung an das Stickstoffatom des Lactamrings in der Verbindung der Formel V findet bei Raumtemperatur oder, falls notwendig, unter Erwärmen, statt. Bei Verwendung des Hydrats der Glyoxylsäure-Verbindung wird Wasser gebildet, das, wenn notwandig, durch Destillation, z.B. azeotrop, oder durch Verwendung eines geeigneten Dehydratationsmittels enfernt wird. Vorzugsweise arbeitet man in Gegenwart eines geeigneten inerten Lösungsmittels oder Lösungsmittelgemisches.

Die Ueberführung einer Hydroxygruppe $X_0$ in eine reaktionsfähige veresterte Hydroxygruppe $X_0$ in einer Verbindung der Formel VI wird durch Behandeln mit einem geeigneten Veresterungsmittel durchgeführt, z.B. mit einem Thionylhalogenid, z.B. -chlorid, vorzugsweise in Gegenwart eines basischen, in erster Linie organischen basischen Mittels, wie eines aliphatischen tertiären Amins, oder einer heterocyclischen Base vom Pyridintyp. Vorzugsweise arbeitet man in Gegenwart eines geeigneten Lösungsmittels, z.B. Dioxan oder Tetrahydrofuran, oder eines Lösungsmittelgemisches, wenn notwendig unter Kühlen, z.B. bei etwa -30° bis etwa 30°C.

Stufe 4

Das Ausgangsmaterial der Formel (II) wird erhalten, indem man eine Verbindung der Formel (VI) mit einer geeigneten Phosphin-Verbindung, wie einem Triniederalkylphosphin, z.B. Tri-n-butyl-phosphin, oder einem Triaryl-phosphin, z.B. Triphenylphosphin, oder mit einer geeigneten Phosphit-Verbindung, wie einem Triniederalkylphosphit, z.B. Triäthylphosphit, oder einem Alkalimetalldiniederalkylphosphit, z.B. -diäthylphosphit, behandelt, und eine gegebenenfalls erhältli chen Verbindung der Formel (II') worin W' für Triphenylmethylthio oder Niederalkanoylthio steht, in eine Verbindung der Formel (II') überführt, worin W' fur den Rest -S-C(=Z)-$(CH_2)_m$-$R_3$ steht.

Die Umsetzung mit der Phosphin- bzw. Phosphit-Verbindung wird vorzugsweise in einem geeigneten inerten Lösungsmittel, wie einem Kohlenwasserstoff, oder einem Aether, oder in einem Lösungsmittelgemisch vorgenommen. Je nach Reaktionsfähigkeit arbeitet man unter Kühlen oder bei erhöhter Temperatur, etwa zwischen -10° und +100°C, bevorzugt bei etwa 20° bis 80°C. Ueblicherweise arbeitet man in Gegenwart eines basischen Mittels, wie einer organischen Base, z.B. eines Amins, oder "Polystyol-Hünigbase", oder einer anorganischen Base, z.B. eines Alkalimetallcarbonats, wobei die primär entstandene Phosphoniumverbindung der Formel

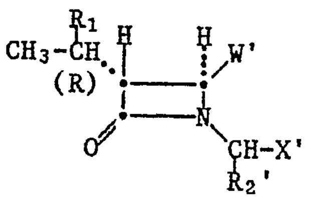

(IIa),

worin X' eine Phosphonogruppe oder eine Phosphoniogruppe zusammen mit einem Anion, je nach Bedeutung des Restes $X_0$ (vgl. Formel VI) z.B. Chlorid, bedeutet, in das Ylid-Ausgangsmaterial der Formel II umgewandelt wird.

Die Einführung des Restes -S-C(=Z)-$(CH_2)_m$-$R_3$ in Verbindungen der Formel II', worin W' für Niederalkanoylthio oder Triphenylmethylthio steht, kann in analoger Weise erfolgen, wie unter Stufe 2 beschrieben.

Man kann das in Reaktionschema 1 beschriebene Verfahren zur Herstellung der Verbindungen der Formeln (II), (III), (V) und (VI), sowie die angegebenen Verfahren zur Herstellung der Endprodukte der Formel (I) auch mit optisch inaktiven Verbindungen durchführen und auf einer beliebigen Verfahrensstufe aus einem erhaltenen Diastereomerengemisch oder Racemat in bekannter Weise die optisch aktiven Verbindungen gemäss der vorliegenden Erfinding isolieren. Die Erfindung betrifft ebenfalls die neuen Ausgangsverbindungen sowie verfahrensgemäss erhältliche neue Zwischenprodukte, wie diejenigen der Formeln (II), (III), ferner (V) und (VI) (W' steht für den Rest -S-C(=Z)-$(CH_2)_m$-$R_3$) sowie die angegebenen Verfahren zu ihrer Herstellung.

Vorzugsweise werden solche Ausgangsverbindungen verwendet und die Reaktionsbedingungen so gewählt, dass man zu den vorstehend als besonders bevorzugt aufgefährten Verbindungen der Formel I gelangt.

Die pharmakologisch verwendbaren Verbindungen der vorliegenden Erfindung können z.B. zur Herstellung von pharmazeutischen Präparaten verwendet werden, welche eine therapeutisch wirksame Menge der Aktivsubstanz zusammen oder im Gemisch mit anorganischen oder organischen, fester oder flüssigen, pharmazeutisch annehmbaren Trägerstoffen enthalten, die sich zur oralen oder zur parenteralen, d.h. z.B. intramuskulären, subcutanen oder intraperitonealen, Verabreichung eignen.

Zur oralen Verabreichung verwendet man Tabletten oder Gelatinekapseln, welche den Wirkstoff zusammen mit Verdünnungsmitteln, z.B. Lactose, Dextrose, Sucrose, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen. Tabletten enthalten ebenfalls Bindemittel, z.B. Magnesiumaluminiumsilikat, Stärken, wie Mais-, Weizen-, Reis-oder Pfeilwurzstärke, Gelatine, Tragacant, Methylcellulose, Natriumcarboxymethylcellulose und/oder Polyvinylpyrrolidon, und, wenn erwünscht, Sprengmittel, z.B. Stärken, Agar, Alginsäure oder Salze davon, wie Natriumalginat, und/oder Brausemischungen oder Adsorptionsmittel, Farbstoffe, Geschmackstoffe oder Süssmittel.

Zur parenteralen Verabreichung eignen sich in erster Linie Infusionslösungen, vorzugsweise isotonische wässrige Lösungen oder Suspensionen, wobei diese z.B. aus lyophilisierten Präparaten, welche die Wirksubstanz allein oder zusammen mit einem Trägermaterial, z.B. Mannit enthalten, vor Gebrauch hergestellt werden können. Solche Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten.

Die vorliegenden pharmazeutischen Präparate, die, wenn erwünscht, weitere pharmakologisch wertvolle Stoffe enthalten können, werden in an sich bekannter Weise, z.B. mittels konventionellen Mischungs-, Lösungs- oder Lyophilisierungsverfahren, hergestellt und enthalten von etwa 0,1 % bis 100 %, insbesondere von etwa 1 % bis etwa 50 %, Lyophilisate bis zu 100 % des Aktivstoffs.

Je nach Art der Infektion und Zustand des infizierten Organismus verwendet man tägliche Dosen (oral oder parenteral) von etwa 100 mg bis etwa 2 g zur Behandlung von Warmblüten (Menschen und Tiere) von etwa 70

kg Gewicht.

Die folgenden Beispiele dienen zur Illustration der Erfindung. Temperaturen werden in Celsiusgraden angegeben.

## Experimenteller Teil

Beispiel 1:

(5R,6S)-2-[4-(Tetrazol-1-yl)-but-1-yl]-6-[(1R)-1-allyloxycarbonyloxyäthyl]-2-penem-3-carbonsäureallylester

Eine Lösung von 2,5 g 2-[(3S,4R)-3-[(1R)-1-Allyloxycarbonyloxyäthyl]-4-[5-(tetrazol-1-yl)-pentanoylthio]-2-oxo-azetidin-1-yl]-2-triphenylphosphoranylidenessigsäureallylester in 500 ml Toluol wird unter Argonatomsphäre 3,5 Stunden bei Rückflusstemperatur gerührt. Dann wird das Lösungsmittel abgedampft und das Rohprodukt durch Chromatographie an Silicagel gereinigt. (Laufmittel Toluol/Aethylacetat 85:15), $R_f$ (Aethylacetat) = 0,55; IR ($CH_2Cl_2$): 1790; 1745; 1705; 1585; 1315; 1240 cm $^{-1}$.

Das Ausgangsmaterial kann wie folgt hergestellt werden:

a) 5-(Tetrazol-1-yl)-valeriansäureäthylester

Eine Lösung von 13,4 g 5-Amino-valeriansäureäthylester-hydrochlorid in 89 ml Essigsäure wird mit 13 g Natriumazid und 59 ml Triäthylorthoformiat versetzt und während 6 Stunden bei 100° gerührt. Das Reaktionsgemisch wird abgekühlt und in Aethylacetat-Waser aufgetrennt. Die organische Phase wird mehrmals mit gesättigter Natriumbicarbonat-Lösung und einmal mit Sole gewaschen und nach Trocknen über Natriumsulfat eingeengt. Nach der Reinigung durch Chromatographie am Silicagel (Laufmittel: Toluol-Aethylacetat 4:1 bis 7:3) erhält man die reine Titelverbindung. $R_f$ (Toluol/Aethylacetat 1:1); 0,32.
IR ($CH_2Cl_2$): 2950; 1725 cm$^{-1}$.

b) 5-(Tetrazol-1-yl)-valeriansäure

2,12 g 5-(Tetrazol-1-yl)-valeriansäure-äthylester werden in 5 ml Methanol gelöst und mit 4,28 ml einer methanolischen NaOH-Lösung (3N) versetzt. Nach 2,5 Stunden Rühren bei Raumtemperatur wird das Lösungsmittel abdestilliert und der Rückstand in Wasser aufgenommen. Nach Waschen mit Aethylacetat wird die wässrige Phase angesäuert (pH 3) und zweimal mit Aethylacetat extrahiert. Nach Trocknen und Eindampfen erhält man die reine Titelverbindung.
NMR (60 MHz, DMSO•$d_6$): δ 12,1 (1H, breit), 9,5 (1H, s), 4,55 (2H,t), 2,3 (2H, t), 1,7 ppm (4H,m).

c) 5-(Tetrazol-1-yl)-valeriansäurechlorid

1 g 5-(Tetrazol-1-yl)-valeriansäure wird in 12 ml absolutem Benzol mit 0,6 ml Thionylchorid und 2 Tropfen DMF 20 Minuten bei Rückflusstemperatur erhitzt. Nach Eindampfen unter vermindertem Druck erhält man die Titelverbindung.
IR (Methylenchlorid): 3145; 1780; 1475 cm$^{-1}$.

d)
2-[(3S,4R)-3-[(1R)-1-Allyloxycarbonyloxyäthyl]-4-[5-(tetrazol-1-yl)-pentanoylthio]-2-oxo-azetidin-1-yl]-2-triphenylphosporanylidenessigsäureallylester

1,74 g Silbersalz des 2-[(3S,4R)-3-[(1R)-1-Allyloxycarbonyloxyäthyl]-4-mercapto-2-oxo-azetidin-1-yl]-2-triphenylphosporanylidenessigsäureallylesters werden in 30 ml Methylenchlorid gelöst, bei 0° mit 0,41 ml Pyridin, 50 mg 4-Dimethylaminopyridin und anschliessend tropfenweise mit einer Lösung von 0,87 g 5-(Tetrazol-1-yl)-valeriansäurechlorid in 15 ml Methylenchlorid versetzt. Nach 30 Minuten Rühren bei 0° wird der Feststoff über Hyflo abfiltriert und das Filtrat wird mit wässriger Natriumbicarbonat-Lösung und dann mit Sole gewaschen. Nach Trocknen über $MgSO_4$ wird das Lösungsmittel abgedampft. Der Rückstand wird durch Chromatographie am Silicagel (Laufmittel Toluol/Aethylacetat: 9:1 bis 1:1) gereinigt. DC (Aethylacetat) $R_f$ = 0,33; IR ($CH_2Cl_2$): 1750; 1690; 1620 cm$^{-1}$.

Beispiel 2: (5R,6S)-2-[4-(Tetrazol-1-yl)-but-1-yl]-6-[(1R)-1-hydroxyäthyl]-2-penem-3-carbonsäure, Natriumsalz

Eine Lösung von 0,9 g (5R,6S)-2-[4-(Tetrazol-1-yl)-but-1-yl]-6-[(1R)-1-allyloxycarbonyloxyäthyl]-2-penem-3-carbonsäureallylester in 34 ml absolutem THF wird mit 95 mg Tetrakis-triphenylphosphinpalladium und 1,13 ml Tributylzinnhydrid versetzt. Nach 30 Minuten Rühren bei Raumtemperatur wird das Gemisch mit 0,58 ml Essigsäure tropfenweise versetzt und weitere 15 Minuten gerührt. Nach dem Konzentrieren am Rotationsverdampfer wird das Gemisch in Wasser-Aethylacetat aufgenommen, mittels $NaHCO_3$ neutral gestellt und zweimal mit Aethylacetat gewaschen. Die wässrige Phase wird am Rotationsverdampfer konzentriert und die Titelsubstanz wird durch anschliessende Chromatographie an Reversed-Phase Opti UPC 12 mit Wasser gereinigt. Die vereinigten Fraktionen werden am Hochvakuum lyophilisiert.
DC (Reversed-Phase Opti UPC 12, Wasser): $R_f$ = 0,5
UV (Wasser): $\lambda_{max}$ = 303 nm

Beispiel 3:
(5R,6S)-2-[5-(Tetrazol-1-yl)-pent-1-yl]-6-[(1R)-1-allyloxycarbonyloxyäthyl]-2-penem-3-carbonsäureallylester

Analog Beispiel 1 werden 1,7 g 2-[(3S,4R)-3-[(1R)-1-Allyloxycarbonyloxyäthyl]-4-[6-(tetrazol-1-yl)-hexanoylthio]-2-oxo-azetidin-1-yl]-2-triphenylphosphoranylideneessigsäureallylester in die Titelverbindung überführt.

IR ($CH_2Cl_2$): 2940; 1790; 1742; 1705; 1580 $cm^{-1}$.

Das Ausgangsmaterial kann wie folgt hergestellt werden:

a) 6-(Tetrazol-1-yl)-hexansäureäthylester

Analog Beispiel 1a) werden 14 g 6-Aminohexansäurehydrochlorid in die Titelverbindung überführt.
IR ($CH_2Cl_2$): 2940; 1725; 1170 $cm^{-1}$.

b) 6-(Tetrazol-1-yl)-hexansäure

Analog Beispiel 1b) werden 8,1 g 6-(Tetrazol-1-yl)-hexansäureäthylester in die Titelverbindung überführt,
$^1H$-NMR (60 MHz, DMSO-$d_6$): $\delta$ = 1,4 (m, 4H); 1,8 (m, 2H); 2,2 (t, 2H); 4,5 (t, 2H); 9,5 (s, 1 H); 12 ppm (br, 1 H).

c)
2-[3S,4R)-3-[(1R)-1-Allyloxycarbonyloxyäthyl]-4-[6-(tetrazol1-yl)-hexanoylthio]-2-oxo-azetidin-1-yl]-2-triphenylphosphoranylidenessigsäureallylester

Eine Suspension von 1,84 g 6-(Tetrazol-1-yl)-hexansäure in 40 ml absolutem Methylenchlorid wird bei Raumtemperatur mit 1,52 ml 1-Dimethylamino-1-chlor-2-methyl-propen versetzt und während 2 Stunden bei Raumtemperatur gerührt. Dieses Reaktionsgemisch wird dann bei 0° einer Lösung von 3,48 g Silbersalz des 2-[(3S,4R)-3-[(1R)-1-Allyloxycarbonyloxyäthyl]-4-mercapto-2-oxoazetidin-1-yl]-2-triphenylphosporanylidenessigsäureallylesters, 0,82 ml Pyridin und 0,1 g 4-Dimethylaminopyridin in 55 ml absolutem Methylenchlorid tropfenweise zugegeben. Nach 30 Minuten wird der Feststoff abfiltriert und das Filtrat mit einer wässrigen Natriumicarbonat Lösung und dann mit Sole gewaschen. Nach Trocknen über Magnesiumsulfat wird eingeengt und der Rückstand durch Chromatographie an Silicagel (Laufmittel Toluol/Aethylacetat 9:1 bis 1:1) gereinigt.

DC (Aethylacetat) $R_f$ = 0,28, IR ($CH_2Cl_2$): 1755; 1695; 1620 $cm^{-1}$.

Beispiel 4: (5R,6S)-2-[5-(Tetrazol-1-yl)-pent-1-yl]-6-[(1R)-1-hydroxyäthyl]-2-penem-3-carbonsäure, Natriumsalz

Analog Beispiel 2 werden 0,58 g (5R,6S)-2-[5-(Tetrazol-1-yl)-pent-1-yl]-6-[(1R)-1-allyloxycarbonyloxäthyl]-2-penem-3-carbonsäureallylester in die Titelverbindung überführt.
UV (Wasser): $\lambda_{max}$ = 302 nm.

Beispiel 5:
(5R,6S)-2-[7-(Tetrazol-1-yl)-hept-1-yl]-6-[(1R)-1-allyloxycarbonyloxyäthyl]-2-penem-3-carbonsäureallylester

Analog Beispiel 1 werden 2,5 g 2-[(3S,4R)-3-[(1R)-1-Allyloxycarbonyloxyäthyl]-4-[8-(tetrazol-1-yl)-octanoylthio]-2-oxoazetidin-1-yl]-2-triphenylphosphoranylidenessigsäureallylester in die Titelverbindung überführt,

IR ($CH_2Cl_2$): 2940; 1790; 1745; 1710; 1580 $cm^{-1}$.

Das Ausgangsmaterial kann wie folgt hergestellt werden:

a) 8-(Tetrazol-1-yl)-octansäureäthylester

Analog Beispiel 1a) werden 19 g 8-Aminooctansäurehydrochlorid in die Titelverbindung überführt.
IR ($CH_2Cl_2$): 2940; 1725; 1168 $cm^{-1}$.

b) 8-(Tetrazol-1-yl)-octansäure

Analog Beispiel 1b) werden 11,3 g 8-(Tetrazol-1-yl)-octansäureäthylester in die Titelverbindung überführt.
$^1H$-NMR (60 MHz, DMSO-$d_6$): $\delta$ = 1,3 - 2,0 (m, 10 H); 2,25 (t, 2H); 4,5 (t, 2H); 9,5 (s, 1H); 8,5 - 10 ppm (br, 1H)

c)
2-[(3S,4R)-[(1R)-1-Allyloxycarbonyloxyäthyl]-4-[8-(tetrazol-1-yl)-octanoylthio]-2-oxo-azetidin-1-yl]-2-triphenylphosphoranylidenessigsäureallylester

Analog Beispiel 3c) werden 2,12 g 8-(Tetrazol-1-yl)-octansäure in die Titelverbindung überführt.
IR ($CH_2Cl_2$): 1755; 1700; 1630 $cm^{-1}$.

Beispiel 6: (5R,6S)-2-[7-(Tetrazol-1-yl)-hept-1-yl]-6-[(1R)-1-hydroxyäthyl]-2-penem-3-carbonsäure, Natriumsalz

Analog Beispiel 2 werden 735 mg (5R,6S)-2-[7-(Tetrazol-1-yl)-hept-1-yl)-6-[(1R)-1-allyloxycarbonyloxyäthyl]-2-penem-3-carbonsäureallylester in die Titelverbindung überführt.
UV (Wasser): $\lambda_{max}$ = 302 nm.

Beispiel 7:
(5R,6S)-2-[10-(Tetrazol-1-yl)-dec-1-yl)-6-[(1R)-1-allyloxycarbonyloxyäthyl]-2-penem-3-carbonsäureallylester

Analog Beispiel 1 werden 2,6 g 2-[(3S,4R)-3-[(1R)-1-Allyloxycarbonyloxyäthyl]-4-[11-(tetrazol-1-yl)-undecanoylthio]-2-oxo-azetidin-1-yl]-2-triphenylphsophoranylidenessigsäureallylester in die Titelverbindung überführt.
IR ($CH_2Cl_2$): 2930; 1790; 1745; 1705; 1635; 1580 $cm^{-1}$.
Das Ausgangsmaterial kann wie folgt hergestellt werden:

a) 11-(Tetrazol-1-yl)-undecansäureäthylester

Analog Beispiel 1a) werden 10,5 g 11-Aminoundecansäure-hydrochlorid in die Titelverbindung überführt.
IR ($CH_2Cl_2$): 2930; 1725; 1165 $cm^{-1}$.

b) 11-(Tetrazol-1-yl)-undecansäure

Analog Beispiel 1b) werden 6,5 g 11-(Tetrazol-1-yl)-undecansäureäthylester in die Titelverbindung überführt.
$^1H$-NMR (60 MHz, DMSO•$d_6$): $\delta$ = 1,3 -2,1 (m, 16H); 2,25 (t, 2H); 4,5 (t, 2H); 9,5 ppm (s, 1H);

c)
2-[(3S,4R)-3-[(1R)-1-Allyloxycarbonyloxyäthyl]-4-[11-(tetrazol-1-yl)-undecanoylthio]-2-oxo-azetidin-1-yl]-2-triphenylphosphoranylidenessigsäureallylester

Analog Beispiel 3c) werden 3,48 g 11-(Tetrazol-1-yl)-undecansäure in die Titelverbindung überführt.
IR ($CH_2Cl_2$); 2930; 1750; 1635 $cm^{-1}$.

Beispiel 8: (5R,6S)-2-[10-(Tetrazol-1-yl)-dec-1-yl]-6-[(1R)-1-hydroxyäthyl]-2-penem-3-carbonsäure, Natriumsalz

Analog Beispiel 2 werden 0,72 g (5R,6S)-2-[10-(Tetrazol-1-yl)-dec-1-yl]-6-[(1R)-1-allyloxycarbonyloxyäthyl]-2-penem-3-carbonsäure-allylester in die Titelverbindung überführt.
UV (Wasser): $\lambda_{max}$ = 302 nm.

Beispiel 9:
(5R,6S)-2-[3-(Tetrazol-1-yl)-prop-1-yl]-6-[(1R)-1-allyloxycarbonyloxyäthyl]-2-penem-3-carbonsäureallylester

Analog Beispiel 1 werden 3,4 g 2-[(3S,4R)-3-[(1R)-1-Allyloxycarbonyloxyäthyl]-4-[4-(tetrazol-1-yl)-butanoylthio]-2-oxo-azetidin-1-yl]-2-triphenylphosphoranylidenessigsäureallylester in die Titelverbindung überführt.
IR ($CH_2Cl_2$); 1790; 1745; 1705; 1580 $cm^{-1}$.
Das Ausgangsmaterial kann wie folgt hergestellt werden:

2-[(3S,4R)-3-[(1R)-1-Allyloxycarbonyloxyäthyl-4-[4-(tetrazol-1-yl)-butanoylthio]-2-oxo-azetidin-1-yl]-2-triphenylphosphoranylidenessigsäureallylester

Analog Beispiel 3c werden 2 g 4-(Tetrazol-1-yl)-buttersäure (vgl. EP 110826) in die Titelverbindung überführt.
IR ($CH_2Cl_2$): 1760; 1690; 1620 $cm^{-1}$.

Beispiel 10: (5R,6S)-2-[3-(Tetrazol-1-yl)-prop-1-yl]-6-[(1R)-1-hydroxyäthyl]-2-penem-3-carbonsäure, Natriumsalz

Analog Beispiel 2 werden 0,56 g (5R,6S)-2-[3-(Tetrazol-1-yl)-prop-1-yl]-6-[(1R)-1-allyloxycarbonyloxyäthyl]-2-penem-3-carbonsäure allylester in die Titelverbindung überführt.
UV (Wasser): $\lambda_{max.}$ = 303 nm.

Beispiel 11:
(5R,6S)-2-(Tetrazol-1-yl)-prop-1-yl]-6-[(1R)-1-hydroxyäthyl]-2-penem-3-carbonsäure-acetoxymethylester

Eine Lösung von 70 mg (5R,6S)-2-[3-(Tetrazol-1-yl)-prop-1-yl]-6-[(1R)-1-hydroxyäthyl]-2-penem-3-carbonsäure, Natriumsalz in 1,5 ml absolutem DMF wird auf 0° gekühlt und mit 40 mg Acetoxymethylbromid versetzt. Nach 35 Minuten erfolgt eine weitere Zugabe von 20 mg Acetoxymethylbromid und das Reaktionsgemisch wird eine weitere Stunde bei Raumtemperatur gerührt. Das Gemisch wird zwischen Wasser und Aethylacetat aufgetrennt und die organische Phase anschliessend zweimal mit Sole gewaschen. Nach Trocknen über Natriumsulfat wird unter vermindertem Druck eingedampft und die Titelverbindung wird durch Chromatographie an Silicagel (Laufmittel Toluol bis Toluol/Aethylacetat: 3:1) gereinigt.
DC (Aethylacetat) $R_f$ = 0,23
IR ($CH_2Cl_2$): 3600; 1790; 1762; 1720; 1580 $cm^{-1}$
UV (Aethanol) $\lambda_{max}$ = 323 nm.

Beispiel 12:
(5R,6S)-2-[3-(Tetrazol-1-yl)-prop-1-yl]-6-[(1R)-1-hydroxyäthyl]-2-penem-3-carbonsäure-pivaloyloxymethylester

Eine Lösung von 70 mg (5R,6S)-2-[3-(Tetrazol-1-yl)-prop-1-yl]-6-[(1R)-1-hydroxyäthyl]-2-penem-3-carbonsäure, Natriumsalz in 1,5 ml absolutem DMF wird auf 0° gekühlt, mit 53 µl Pivaloyloxymethyljodid versetzt und 70 Minuten bei gleicher Temperatur gerührt. Das Reaktionsgemisch wird mit Aethylacetat verdünnt, dreimal mit Sole gewaschen, über Natriumsulfat getrocknet und eingeengt. Nach Trocknung im Hochvakuum wird die Titelverbindung durch Chromatographie an Silicagel (Laufmittel: Toluol bis Toluol/Aethylacetat: 3:1) gereinigt.
DC (Aethylacetat) $R_f = 0,28$.
IR ($CH_2Cl_2$): 3600; 1790; 1760; 1720; 1580 $cm^{-1}$.
UV (EtOH): $\lambda_{max} = 322$ nm.

Beispiel 13:
(5R,6S)-2-[3-(Tetrazol-1-yl)-prop-1-yl]-6-[(1R)-1-hydroxyäthyl]-2-penem-3-carbonsäure-1-äthoxycarbonyloxyäthylester

1,2 g Natriumjodid werden in 3,7 ml Aceton gelöst und mit 0,275 ml Aethyl-1-chloräthylcarbonat versetzt. Das Gemisch wird bei Raumtemperatur während 3 Stunden gerührt. Anschliessend wird die Lösung auf 15 ml Methylenchlorid getropft und von den ausgefallenen anorganischen Salzen abfiltriert. Die Methylenchloridlösung wird bis auf 2 ml eingeengt und bei 0° zu einer Lösung von 0,347 g (1 mMol) (5R,6S)-2-[3-(Tetrazol-1-yl)-prop-1-yl]-6-[(1R)-1-hydroxyäthyl]-2-penem-3-carbonsäure in 4 ml Dimethylacetamid gegeben. Dann wird während 3 Stunden bei 0° gerührt, anschliessend mit Aethylacetat verdünnt und dreimal mit Wasser gewaschen. Die organischen Phasen werden über Natriumsulfat getrocknet und am Rotationsverdampfer eingeengt. Das Rohprodukt wird an 10 g Silicagel mit dem Laufmittel Aethylacetat gereinigt. Man erhält die Titelverbindung als weissen Schaum. IR-Spektrum (Methylenchlorid): 3590; 1790; 1748; 1720; 1665 $cm^{-1}$.

Beispiel 14:

Trockenampullen oder Vials, enthaltend 0,5 g (5R,6S)-2-[3-(Tetrazol-1-yl)-prop-1-yl]-6-[(1R)-1-hydroxyäthyl]-2-penem-3-carbonsäure, Natriumsalz als Wirksubstanz, werden wie folgt hergestellt:

Zusammensetzung (für 1 Ampulle oder Vial):
Wirksubstanz 0,5 g
Mannit 0,5 g

Eine sterile wässrige Lösung der Wirksubstanz und des Mannits wird unter aseptischen Bedingungen in 5 ml-Ampullen oder 5 ml-Vials der Gefriertrocknung unterworfen und die Ampullen bzw. Vials verschlossen und geprüft.

Anstelle des obengenannten Wirkstoffs kann auch dieselbe Menge eines anderen Wirkstoffs der vorangehenden Beispiele verwendet werden.

**Patentansprüche**

1. Verbindungen der Formel

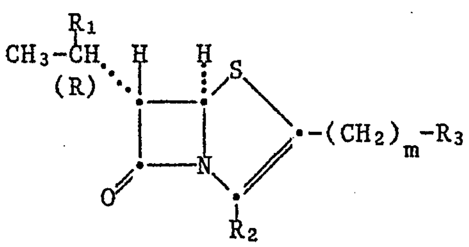

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m 3 oder 4 bedeutet, und Salze solcher Verbindungen.

2. Verbindungen der Formel I gemäss Anspruch 1, worin $R_1$ Hydroxy oder geschütztes Hydroxy bedeutet, $R_2$ Carboxyl, unter physiologischen Bedingungen spaltbares verestertes Carboxyl oder geschütztes Carboxyl ist, $R_3$ einen über ein Ringstickstoffatom gebundenen monocyclischen 5-gliedrigen Heteroarylrest mit 1 bis 4 Ringstickstoffatomen darstellt, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Carbamoylniederalkyl, Carbamoyloxyniederalkyl, Halogenniederalkyl, Aminoniederalkyl, Niederalkylaminoniederalkyl, Diniederalkylaminoniederalkyl, Niederalkanoylaminoniederalkyl, Amino-carboxy-niederalkyl, Sulfoniederalkyl, Amino, Niederalkylamino, Diniederalkylamino, Niederalkanoylamino, Carboxyl, Niederalkoxycarbonyl, Carbamoyl, Cyano, Sulfo, Sulfamoyl und/oder gegebenenfalls durch Niederalkyl, Niederalkoxy und/oder Halogen substituiertes Phenyl substituiert ist, und m 3 oder 4 bedeutet, und Salze von Verbindungen der Formel I.

3. Verbindungen der Formel I gemäss Anspruch 1, worin $R_1$ Hydroxy ist, $R_2$ Carboxyl oder unter

physiologischen Bedingungen spaltbares verestertes Carboxyl bedeutet, $R_3$ unsubstituiertes oder durch Niederalkyl substituiertes 1-Pyrrolyl, unsubstituiertes oder durch Niederalkyl oder Halogen substituiertes 1-Pyrazolyl, unsubstituiertes oder durch Niederalkyl, Niederalkanoyloxyniederalkyl, Halogenniederalkyl, Aminoniederalkyl, Niederalkanoylaminoniederalkyl und/oder Niederalkoxy substituiertes 1-Imidazolyl, unsubstituiertes oder durch Niederalkyl oder Niederalkoxycarbonyl substituiertes 1,2,4- oder 1,3,4-Triazol-1-yl, oder unsubstituiertes oder durch Niederalkyl substituiertes 1- oder 2-Tetrazolyl bedeutet und m 3 oder 4 ist, und Salze von Verbindungen der Formel I.

4. Verbindungen der Formel I gemäss Anspruch 1, worin $R_1$ Hydroxy ist, $R_2$ Carboxyl oder unter physiologischen Bedingungen spaltbares verestertes Carboxyl bedeutet, $R_3$ 1-Tetrazolyl ist und m 3 oder 4 ist, und Salze von Verbindungen der Formel I.

5. Verbindungen der Formel I gemäss Anspruch 1, worin $R_2$ eine unter physiologischen Bedingungen spaltbare veresterte Carboxylgruppe darstellt.

6. (5R,6S)-2-[4-(Tetrazol-1-yl)-but-1-yl]-6-[(1R)-1-hydroxyäthyl]-2-penem-3-carbonsäure und pharmazeutisch annehmbare Salze davon gemäss Anspruch 1.

7. (5R,6S)-2-[3-(Tetrazol-1-yl)-prop-1-yl]-6-[(1R)-1-hydroxyäthyl]-2-penem-3-carbonsäure und pharmazeutisch annehmbare Salze davon gemäss Anspruch 1.

8. (5R,6S)-2-[3-(Tetrazol-1-yl)-prop-1-yl]-6-[(1R)-1-hydroxyäthyl]-2-penem-3-carbonsäure-acetoxymethylester gemäss Anspruch 1.

9. (5R,6S)-2-[3-(Tetrazol-1-yl)-prop-1-yl]-6-[(1R)-1-hydroxyäthyl]-2-penem-3-carbonsäure-pivaloyloxymethylester gemäss Anspruch 1.

10. Eine Verbindung gemäss Anspruch 1 und ihre pharmazeutisch annehmbaren Salze zur Anwendung in einen Verfahren zur therapeutischen Behandlung des menschlichen und tierischen Körpers.

11. Pharmazeutisches Präparat enthaltend eine Verbindung der Formel I gemäss Anspruch 1 oder ein pharmazeutisch annehmbares Salz davon.

12. Verwendung von Verbindungen der Formel I gemäss Anspruch 1 und von pharmazeutisch annehmbaren Salzen solcher Verbindungen zur Herstellung von pharmazeutischen Präparaten.

13. Verfahren zur Herstellung von Verbindungen der Formel I gemäss Anspruch 1, dadurch gekennzeichnet, dass man eine Ylid-Verbindung der Formel

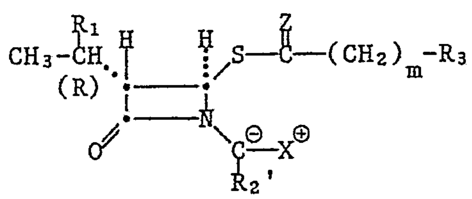

(II),

worin $R_1$,m und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

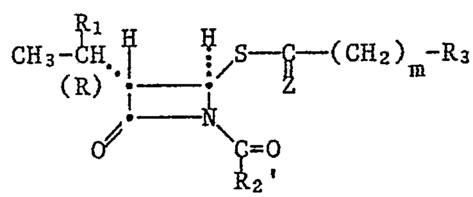

(III),

worin $R_1$, m und $R_3$ die unter Formel I und $R_2'$und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppen in die freien funktionellen Gruppen überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in eine Salz oder ein erhältliches Salz in die freie Verbindung oder in ein anderes Salz überführt.

14. Die nach dem Verfahren gemäss Anspruch 13 erhältlichen Verbindungen.

15. Verbindungen der Formel

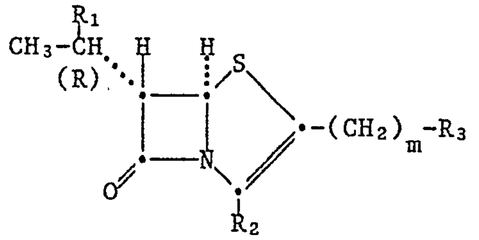

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m eine ganze Zahl von 5 bis 12 bedeutet, und Salze solcher Verbindungen.

16. Verbindungen der Formel I gemäss Anspruch 15, worin $R_1$ Hydroxy oder geschütztes Hydroxy bedeutet, $R_2$ Carboxyl, unter physiologischen Bedingungen spaltbares verestertes Carboxyl oder geschütztes Carboxyl ist, $R_3$ einen über ein Ringstickstoffatom gebundenen monocyclischen 5-gliedrigen Heteroarylrest mit 1 bis 4 Ringstickstoffatomen darstellt, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Carbamoylniederalkyl, Carbamoyloxyniederalkyl, Halogenniederalkyl, Aminonieder alkyl, Niederalkylaminoniederalkyl, Diniederalkylaminoniederalkyl, Niederalkanoylaminoniederalkyl, Amino-carboxy-niederalkyl, Sulfoniederalkyl, Amino, Niederalkylamino, Diniederalkylamino, Niederalkanoylamino, Carboxyl, Niederalkoxycarbonyl, Carbamoyl, Cyano, Sulfo, Sulfamoyl und/oder gegebenenfalls durch Niederalkyl, Niederalkoxy und/oder Halogen substituiertes Phenyl substituiert ist, und m eine ganze Zahl von 5 bis 12 bedeutet, und Salze von Verbindungen der Formel I.

17. Verbindungen der Formel I gemäss Anspruch 15, worin $R_1$ Hydroxyist, $R_2$ Carboxyl oder unter physiologischen Bedingungen spaltbares verestertes Carboxyl bedeutet, $R_3$ unsubstituiertes oder durch Niederalkyl substituiertes 1-Pyrrolyl, unsubstituiertes oder durch Niederalkyl oder Halogen substituiertes 1-Pyrazolyl, unsubstituiertes oder durch Niederalkyl, Niederalkanoyloxyniederalkyl, Halogenniederalkyl, Aminoniederalkyl, Niederalkanoylaminoniederalkyl und/oder Niederalkoxy substituiertes 1-Imidazolyl, unsubstituiertes oder durch Niederalkyl oder Niederalkoxycarbonyl substituiertes 1,2,4- oder 1,3,4-Triazol-1-yl, oder unsubstituiertes oder durch Niederalkyl substituiertes 1- oder 2-Tetrazolyl bedeutet und m eine ganze Zahl von 5 bis 10 ist, und Salze von Verbindungen der Formel I.

18. Verbindung der Formel I gemäss Anmspruch 15, worin $R_1$ Hydroxy ist, $R_2$ Carboxyl oder unter physiologischen Bedingungen spaltbares verestertes Carboxyl bedeutet, $R_3$ 1-Tetrazolyl ist und m eine ganze Zahl von 5 bis 8 ist, und Salze von Verbindungen der Formel I.

19. Verbindungen der Formel I gemäss Anspruch 15, worin $R_2$ eine unter physiologischen Bedingungen spaltbare veresterte Carboxylgruppe darstellt.

20. (5R,6S)-2-[5-(Tetrazol-1-yl)-pent-1-yl]-6-[(1R)-1-hydroxyäthyl]-2-penem-3-carbonsäure und pharmazeutisch annehmbare Salze davon gemäss Anspruch 15.

21. (5R,6S)-[7-(Tetrazol-1-yl)-hept-1-yl-6-[(1R)-1-hydroxyäthyl]-2-penem-3-carbonsäure und pharmazeutisch annehmbare Salze davon gemäss Anspruch 15.

22. (5R,6S)-2-[10-(Tetrazol-1-yl)-dec-1-yl]-6-[(1R)-1-hydroxyäthyl]-2-penem-3-carbonsäure und pharmazeutisch annehmbare Salze davon gemäss Anspruch 15.

23. Eine Verbindung gemäss Anspruch 15 und ihre pharmazeutisch annehmbaren Salze zur Anwendung in einem Verfahren zur therapeutischen Behandlung des menschlichen und tierischen Körpers.

24. Pharmazeutisches Präparat enthaltend eine Verbindung der Formel I gemäss Anspruch 15 oder ein pharmazeutisch annehmbares Salz davon.

25. Verwendung von Verbindungen der Formel I gemäss Anspruch 15 und von pharmazeutisch annehmbaren Salzen solcher Verbindungen zur Herstellung von pharmazeutischen Präparaten.

26. Verfahren zur Herstellung von Verbindungen der Formel I gemäss Anspruch 15, dadurch gekennzeichnet, dass man eine Ylid-Verbindung der Formel

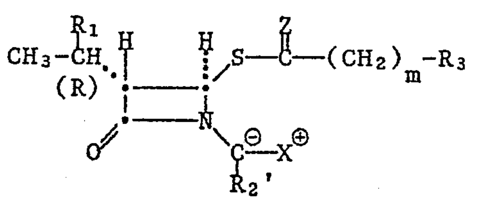

(II),

worin $R_1$,m und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

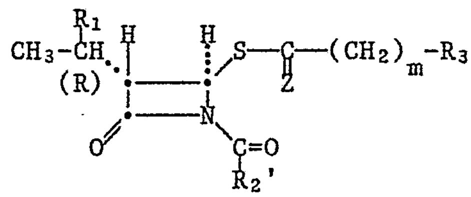

(III),

worin $R_1$, m und $R_3$ die unter Formel I und $R_2'$ und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppe in die freien funktionellen Gruppen überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in ein Salz oder ein erhältliches Salz in die freie Verbindung oder in ein anderes Salz überführt.

27. Die nach dem Verfahren gemäss Anspruch 26 erhältlichen Verbindungen.

Patentansprüche für die folgenden Vertragstaaten: ES, GR

1. Verfahren zur Herstellung von Verbindungen der Formel

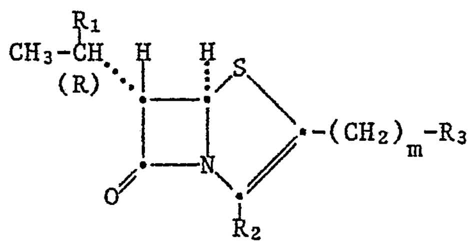

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m 3 oder 4 bedeutet, und Salzen solcher Verbindungen, dadurch gekennzeichnet, dass man eine Ylid-Verbindung der Formel

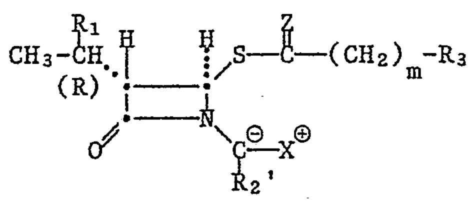

(II),

worin $R_1$,m und $R_3$ die unter Formel I angegebenen Bedeutungen haben, $R_2'$ eine geschützte Carboxylgruppe darstellt, Z Sauerstoff oder Schwefel bedeutet und $X^{\oplus}$ entweder eine dreifach substituierte Phosphoniogruppe oder eine zweifach veresterte Phosphonogruppe zusammen mit einem Kation darstellt, ringschliesst, oder eine Verbindung der Formel

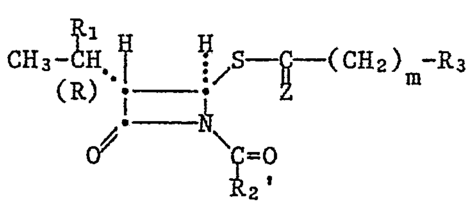

(III),

worin $R_1$, m und $R_3$ die unter Formel I und $R_2'$und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppen in die freien funktionellen Gruppen überführt, und/oder, wenn erwünscht, in einer erhältlichen Verbindung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in eine Salz oder ein erhältliches Salz in die freie Verbindung oder in ein anderes Salz überführt.

2. Verfahren zur Herstellung von Verbindungen der Formel I gemäss Anspruch 1, worin $R_1$ Hydroxy oder geschütztes Hydroxy bedeutet, $R_2$ Carboxyl, unter physiologischen Bedingungen spaltbares verestertes Carboxyl oder geschütztes Carboxyl ist, $R_3$ einen über ein Ringstickstoffatom gebundenen monocyclischen 5-gliedrigen Heteroarylrest mit 1 bis 4 Ringstickstoffatomen darstellt, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Carbamoylniederalkyl, Carbamoyloxyniederalkyl, Halogenniederalkyl, Aminoniederalkyl, Niederalkylaminoniederalkyl, Diniederalkylaminoniederalkyl, Niederalkanoylaminoniederalkyl, Amino-carboxy-niederalkyl, Sulfoniederalkyl, Amino, Niederalkylamino, Diniederalkylamino, Niederalkanoylamino, Carboxyl, Niederalkoxycarbonyl, Carbamoyl, Cyano, Sulfo, Sulfamoyl und/oder gegebenenfalls durch Niederalkyl, Niederalkoxy und/oder Halogen substituiertes Phenyl substituiert ist, und m 3 oder 4 bedeutet, und Salzen davon.

3. Verfahren zur Herstellung von Verbindungen der Formel I gemäss Anspruch 1, worin $R_1$ Hydroxy ist, $R_2$ Carboxyl oder unter physiologischen Bedingungen spaltbares verestertes Carboxyl bedeutet, $R_3$ unsubstituiertes oder durch Niederalkyl substituiertes 1-Pyrrolyl, unsubstituiertes oder durch Niederalkyl oder Halogen substituiertes 1-Pyrazolyl, unsubstituiertes oder durch Niederalkyl, Niederalkanoyloxyniederalkyl, Halogenniederalkyl, Aminoniederalkyl, Niederalkanoylaminoniederalkyl und/oder Niederalkoxy substituiertes 1-Imidazolyl, unsubstituiertes oder durch Niederalkyl oder Niederalkoxycarbonyl unsubstituiertes 1,2,4- oder 1,3,4-Triazol-1-yl, oder unsubstituiertes oder durch Niederalkyl substituiertes 1- oder 2-Tetrazolyl bedeutet und m 3 oder 4 ist, und Salzen davon.

4. Verfahren zur Herstellung von Verbindungen der Formel I gemäss Anspruch 1, worin $R_1$ Hydroxy ist, $R_2$ Carboxyl oder unter physiologischen Bedingungen spaltbares verestertes Carboxyl bedeutet, $R_3$ 1-Tetrazolyl ist und m 3 oder 4 ist, und Salzen davon.

5. Verfahren zur Herstellung von Verbindungen der Formel I gemäss Anspruch 1, worin $R_2$ eine unter physiologischen Bedingungen spaltbare veresterte Carboxylgruppe darstellt.

6. Verfahren gemäss Anspruch 1 zur Herstellung von (5R,6S)-2-[4-(Tetrazol-1-yl)-but-1-yl]-6-[(1R)-1-hydroxyäthyl]-2-penem-3-carbonsäure und pharmazeutisch annehmbare Salze davon.

7. Verfahren gemäss Anspruch 1 zur Herstellung von (5R,6S)-2-[3-(Tetrazol-1-yl)-prop-1-yl]-6-[(1R)-1-hydroxyäthyl]-2-penem-3-carbonsäure und pharmazeutisch annehmbaren Salzen davon.

8. Verfahren gemäss Anspruch 1 zur Herstellung von (5R,6S)-2-[3-(Tetrazol-1-yl)-prop-1-yl]-6-[(1R)-1-hydroxyäthyl]-2-penem-3-carbonsäure-acetoxymethylester.

9. Verfahren gemäss Anspruch 1 zur Herstellung von (5R,6S)-2-[3-(Tetrazol-1-yl)-prop-1-yl]-6-[(1R)-1-hydroxyäthyl]-2-penem-3-carbonsäure-pivaloyloxymethylester.

10. Verfahren zur Herstellung von Verbindungen der Formel

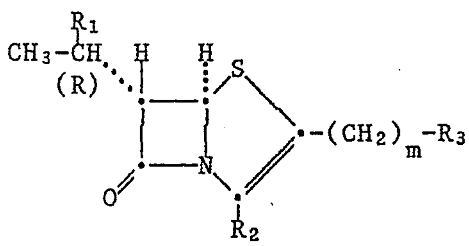

(I),

worin $R_1$ Hydroxy oder geschütztes Hydroxy bedeutet, $R_2$ Carboxyl oder funktionell abgewandeltes Carboxyl ist, $R_3$ einen über ein Ringstickstoffatom gebundenen monocyclischen Heteroaryl-Rest mit 1 bis 4 Ringstickstoffatomen darstellt und m eine ganze Zahl von 5 bis 12 bedeutet, und Salzen solcher Verbindungen, dadurch gekennzeichnet, dass man eine Ylid-Verbindung der Formel

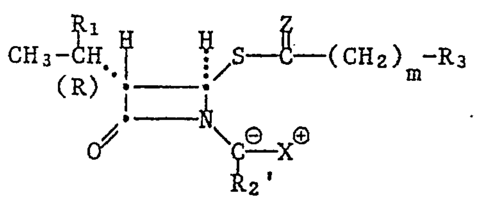

(II),

worin $R_1$,m und $R_3$ die unter Formel I angegebenen Bedeutungen haben, $R_2'$ eine geschützte Carboxylgruppe darstellt, Z Sauerstoff oder Schwefel bedeutet und $X^\oplus$ entweder eine dreifach substituierte Phosphoniogruppe oder eine zweifach veresterte Phosphonogruppe zusammen mit einem Kation darstellt, ringschliesst, oder eine Verbindung der Formel

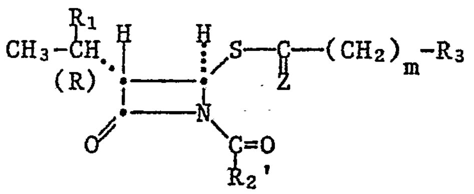

(III),

worin $R_1$, m und $R_3$ die unter Formel I und $R_2'$und Z die unter Formel II angegebenen Bedeutungen haben, mit einer organischen Verbindung des dreiwertigen Phosphors behandelt, und, wenn erwünscht oder notwendig, in einer erhältlichen Verbindung der Formel I geschützte funktionelle Gruppen in die freien funktionellen Gruppen überführt, und/oder, wenn erwünscht, in einer erhältlichen Verbin dung der Formel I eine freie Carboxylgruppe $R_2$ in eine unter physiologischen Bedingungen spaltbare veresterte Carboxylgruppe überführt, und/oder, wenn erwünscht, in einer erhältlichen Verbindung der Formel I einen Rest $R_3$ in einen anderen Rest $R_3$ überführt, und/oder, wenn erwünscht, eine erhältliche Verbindung mit salzbildender Gruppe in eine Salz oder ein erhältliches Salz in die freie Verbindung oder in ein anderes Salz überführt.

11. Verfahren zur Herstellung von Verbindungen der Formel I gemäss Anspruch 10, worin $R_1$ Hydroxy oder geschütztes Hydroxy bedeutet, $R_2$ Carboxyl, unter physiologischen Bedingungen spaltbares verestertes Carboxyl oder geschütztes Carboxyl ist, $R_3$ einen über ein Ringstickstoffatom gebundenen monocyclischen 5-gliedrigen Heteroarylrest mit 1 bis 4 Ringstickstoffatomen darstellt, welcher unsubstituiert oder durch Hydroxy, Niederalkoxy, Niederalkanoyloxy, Halogen, Mercapto, Niederalkylthio, Phenylthio, Niederalkyl, Hydroxyniederalkyl, Niederalkanoyloxyniederalkyl, Carboxyniederalkyl, Carbamoylniederalkyl, Carbamoyloxyniederalkyl, Halogenniederalkyl, Aminoniederalkyl, Niederalkylaminoniederalkyl, Diniederalkylaminoniederalkyl, Niederalkanoylaminoniederalkyl, Amino-carboxy-niederalkyl, Sulfoniederalkyl, Amino, Niederalkylamino, Diniederalkylamino, Niederalkanoylamino, Carboxyl, Niederalkoxycarbonyl, Carbamoyl, Cyano, Sulfo, Sulfamoyl und/oder gegebenenfalls durch Niederalkyl, Niederalkoxy und/oder Halogen substituiertes Phenyl substituiert ist, und m eine ganze Zahl von 5 bis 12 bedeutet, und Salzen davon.

12. Verfahren zur Herstellung von Verbindungen der Formel I gemäss Anspruch 10, worin $R_1$ Hydroxy ist, $R_2$ Carboxyl oder unter physiologischen Bedingungen spaltbares verestertes Carboxyl bedeutet, $R_3$ unsubstituiertes oder durch Niederalkyl substituiertes 1-Pyrrolyl, unsubstituiertes oder durch Niederalkyl oder Halogen substituiertes 1-Pyrazolyl, unsubstituiertes oder durch Niederalkyl, Niederalkanoyloxyniederalkyl, Halogenniederalkyl, Aminoniederalkyl, Niederalkanoylaminoniederalkyl und/oder Niederalkoxy substituiertes 1-Imidazolyl, unsubstituiertes oder durch Niederalkyl oder Niederalkoxycarbonyl unsubstituiertes 1,2,4- oder 1,3,4-Triazol-1-yl, oder unsubstituiertes oder durch Niederalkyl substituiertes 1- oder 2-Tetrazolyl bedeutet und m eine ganze Zahl von 5 bis 10 ist, und Salzen davon.

13. Verfahren zur Herstellung von Verbindungen der Formel I gemäss anspruch 10, worin $R_1$ Hydroxy ist, $R_2$ Carboxyl oder unter physiologischen Bedingungen spaltbares verestertes Carboxyl bedeutet, $R_3$ 1-Tetrazolyl ist und m eine ganze Zahl von 5 bis 8 ist, und Salzen davon.

14. Verfahren zur Herstellung von Verbindungen der Formel I gemäss Anspruch 10, worin $R_2$ eine unter physiologischen Bedingungen spaltbare veresterte Carboxylgruppe darstellt.

15. Verfahren gemäss Anspruch 10 zur Herstellung von (5R,6S)-2-[5-(Tetrazol-1-yl)-pent-1-yl]-6-[(1R)-1-hydroxyäthyl]-2-penem-3-carbonsäure und pharmazeutisch annehmbaren Salzen davon.

16. Verfahren gemäss Anspruch 10 zur Herstellung von (5R,6S)-2-[7-(Tetrazol-1-yl)-hept-1-yl]-6-[(1R)-1-hydroxyäthyl]-2-penem-3-carbonsäure und pharmazeutisch annehmbaren Salzen davon.

17. Verfahren gemäss Anspruch 10 zur Herstellung von (5R,6S)-2-[10-(Tetrazol-1-yl)-dec-1-yl]-6-[(1R)-1-hydroxyäthyl]-2-penem-3-carbonsäure und pharmazeutisch annehmbaren Salzen davon.

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

Nummer der Anmeldung
EP 88 81 0006

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A - 0 110 826 (CIBA-GEIGY)<br>* Ansprüche * | 1-9, 12-14 | C 07 D 499/00<br>A 61 K 31/43//<br>C 07 D 257/04<br>C 07 F 9/65 |
| X | EP - A - 0 201 206 (FARMITALIA CARLO ERBA)<br>* Spalten 11,19,20; Spalten 57,58; Spalten 65-82; Ansprüche * | 1-5, 12-14 | |

RECHERCHIERTE SACHGEBIETE (Int. Cl.4)

C 07 D 499/00
A 61 K 31/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-9,11-22,24-27

Unvollständig recherchierte Patentansprüche:

Nicht recherchierte Patentansprüche: 10,23

Grund für die Beschränkung der Recherche:

Verfahren zur chirurgischen oder therapeutischen Behandlung des menschlichen oder tierischen Körpers (siehe Art. 52(4) des Europäischen Patentübereinkommens).

| Recherchenort | Abschlußdatum der Recherche | Prüfer |
|---|---|---|
| DEN HAAG | 18-03-1988 | CHOULY |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82